(19) **Europäisches Patentamt** / **European Patent Office** / **Office européen des brevets**

(11) **EP 4 201 331 B1**

(12) **EUROPEAN PATENT SPECIFICATION**

(45) Date of publication and mention of the grant of the patent:
**11.06.2025 Bulletin 2025/24**

(21) Application number: **21866101.5**

(22) Date of filing: **13.09.2021**

(51) International Patent Classification (IPC):
*A61B 6/00* (2024.01)    *G06T 7/70* (2017.01)
*G06T 15/08* (2011.01)    *A61B 6/03* (2006.01)
*A61B 6/02* (2006.01)    *A61B 6/50* (2024.01)

(52) Cooperative Patent Classification (CPC):
**A61B 6/487; A61B 6/5229;** A61B 6/025;
A61B 6/032; A61B 6/037; A61B 6/4441;
A61B 6/481; A61B 6/482; A61B 6/502; A61B 6/504

(86) International application number:
**PCT/CN2021/118006**

(87) International publication number:
**WO 2022/053049 (17.03.2022 Gazette 2022/11)**

(54) **DYNAMIC PERSPECTIVE METHOD, APPARATUS AND SYSTEM FOR C-SHAPED ARM EQUIPMENT**

DYNAMISCHES PERSPEKTIVENVERFAHREN, VORRICHTUNG UND SYSTEM FÜR EINE C-FÖRMIGE ARMAUSRÜSTUNG

PROCÉDÉ, APPAREIL ET SYSTÈME DE PERSPECTIVE DYNAMIQUE POUR ÉQUIPEMENT DE BRAS EN FORME DE C

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.09.2020 CN 202010957835**
**24.09.2020 CN 202011019324**
**24.09.2020 CN 202011017481**
**24.09.2020 CN 202011019279**

(43) Date of publication of application:
**28.06.2023 Bulletin 2023/26**

(73) Proprietor: **Shanghai United Imaging Healthcare Co., Ltd.**
**Shanghai 201807 (CN)**

(72) Inventors:
• **SUN, Biao**
**Shanghai 201807 (CN)**
• **XIANG, Jun**
**Shanghai 201807 (CN)**
• **WANG, Zhenwei**
**Shanghai 201807 (CN)**
• **WANG, Weiyi**
**Shanghai 201807 (CN)**
• **ZHANG, Na**
**Shanghai 201807 (CN)**
• **FENG, Juan**
**Shanghai 201807 (CN)**

(74) Representative: **Wang, Bo**
**Panovision IP**
**Ebersberger Straße 3**
**85570 Markt Schwaben (DE)**

(56) References cited:
CN-A- 101 126 725    CN-A- 102 068 268
CN-A- 107 146 266    CN-A- 111 261 265
CN-A- 111 973 209    CN-A- 112 057 094
CN-A- 112 150 543    CN-A- 112 150 600
US-A1- 2004 215 071    US-A1- 2016 287 201
US-A1- 2020 218 922    US-A1- 2020 218 922

**Description**

**TECHNICAL FIELD**

The present disclosure relates to the field of image processing technology, and in particular, to a method, device and system for a dynamic fluoroscopy of a C-shaped arm device.

**BACKGROUND**

The radiological devices (e.g., a DSA device, a DR device, an X-ray machine, a mammography machine, etc.) photograph and/or treat a subject by emitting radiation (e.g., X-rays). When performing a surgery/diagnosis, if a medical worker may dynamically know the changes (e.g., movement) of the subject's lesion and/or tissue and organ, he/she can obtain a diagnostic result or perform a clinical operation more quickly and accurately.

Therefore, there is a need to provide a method and system for a dynamic fluoroscopy to better assist in diagnosis/therapy.

US2016287201A1 discloses a method for imaging and positioning of a medical imaging device, comprising: obtaining a virtual human body model corresponding to an imaging object and first position information corresponding to a user operation instruction ; determining, based on the first position information, an internal human body image corresponding to the first position information in the virtual human body model and displaying the internal human body image. Said document also discloses the corresponding device.

**SUMMARY**

In view of this, the present disclosure provides a method, device and system for a dynamic fluoroscopy of a C-shaped arm device, so that medical personnel can dynamically understand the changes and movements of the lesions and/or various tissues and organs of the subject, and thus obtain diagnostic results or perform clinical operations more quickly and accurately.

In a first aspect, embodiments of the present disclosure provide a method for a dynamic fluoroscopy of a C-shaped arm device, comprising: photographing a subject during a photography cycle, obtaining during the photography first fluoroscopic data of a radiation source irradiating the subject at a first energy, and obtaining second fluoroscopic data of the radiation source irradiating the subject at a second energy different from the first energy; performing the photography of the subject in multiple successive photography cycles; displaying a dynamic image of the subject based on the first fluoroscopic data and the second fluoroscopic data obtained in each of the multiple successive photography cycles.

In a second aspect, embodiments of the present disclosure provide an imaging positioning method for a medical imaging device, the method comprising:

> obtaining a virtual human body model corresponding to an imaging object and obtaining first position information corresponding to a user operation instruction;
> determining, based on the first position information, an internal human body image corresponding to the first position information in the virtual human body model, and displaying the internal human body image;
> determining, based on the first position information, a target imaging position corresponding to the medical imaging device, if the internal human body image corresponding to the first position information corresponds to a target photography position;
> if the internal human body image corresponding to the first position information does not correspond to the target photography position, proceeding to obtain a second position information different from the first position information and determining the target imaging position corresponding to the medical imaging device based on the internal human body image corresponding to the second position information.

The embodiments of the present disclosure determine the internal human body image corresponding to the first position information input by the user based on the virtual human body model, and obtain the target imaging position corresponding to the medical imaging device by judging whether the internal human body image corresponds to the target shooting position, solving the problem of radiation damage to the human body during the imaging operation, allowing the user to set any parameters in the process of imaging and positioning without causing any damage to the human body, which in turn also ensures the positioning accuracy.

In a third aspect, embodiments of the present disclosure provide an image display method, the method comprising:

> obtaining a first blood vessel image of an imaging object obtained by the angiography device based on a first horizon-of-view parameter, and displaying the first blood vessel image;

determining a second horizon-of-view parameter based on a received parameter adjustment instruction and the first horizon-of-view parameter; wherein the parameter adjustment instruction includes a scaling adjustment ratio;

determining a second blood vessel image based on the second horizon-of-view parameter and simultaneously displaying the second blood vessel image and the first blood vessel image.

By determining the second blood vessel image based on the received parameter adjustment instruction while displaying the first blood vessel image, and displaying the second blood vessel image simultaneously with the first blood vessel image, the embodiments of the present disclosure solve the problem of repeatedly switching the display of scanned images of different horizon-of-views, reduce the number of repeated imaging and the amount of radiation, improve the service life of the angiography device, and in turn also improve the diagnostic efficiency of angiography and reduce the error of the diagnostic results of angiography caused by repeated switching.

In a fourth aspect, embodiments of the present disclosure provide a method for generating a volume reconstruction image, comprising:

obtaining projection data at each scanning angle;

constructing a target volume coordinate system based on an initial volume coordinate system and a desired reconstruction direction; and

reconstructing the projection data according to the desired reconstruction direction under the target volume coordinate system to generate a target volume reconstruction image.

This embodiment provides a technical solution to generate a target volume reconstruction image by obtaining projection data at each scanning angle, constructing a target volume coordinate system based on an initial volume coordinate system and a desired reconstruction direction, and reconstructing the projection data according to the desired reconstruction direction under the target volume coordinate system. The problem that only a better image can be obtained in the direction parallel to the detector but not in other directions in the prior art is solved. By setting different desired reconstruction directions, it achieves the purpose of reconstructing in different desired reconstruction directions and obtaining a better resolution volume reconstruction image in each direction, which facilitates the user to effectively analyze the volume reconstruction images in multiple reconstruction directions and perform target positioning.

In the fifth aspect, embodiments of the present disclosure provide a system for a dynamic fluoroscopy of a C-shaped arm device, comprising a photography module and a display module; the photography module configured to photograph a subject during a photography cycle, obtain, during the photography cycle, first fluoroscopic data of a radiation source irradiating the subject at a first energy and second fluoroscopic data of the radiation source irradiating the subject at a second energy different from the first energy; the photography module further configured to perform the photography of the subject in multiple successive photography cycles; the display module configured to display a dynamic image of the subject based on the first fluoroscopic data and the second fluoroscopic data obtained in each of the multiple successive photography cycles.

In a sixth aspect, embodiments of the present disclosure provide an imaging positioning device for a medical imaging device, the device comprising:

a virtual human body model acquisition module, configured to obtain a virtual human body model corresponding to an imaging object and obtain first position information corresponding to a user operation instruction;

an internal human body image display module, configured to determine, based on the first position information, an internal human body image corresponding to the first position information in the virtual human body model, and display the internal human body image;

a first target imaging position determination module, configured to determine, based on the first position information, a target imaging position corresponding to the medical imaging device, if the internal human body image corresponding to the first position information corresponds to a target photography position;

a second target imaging position determination module, configured to proceed to obtain a second position information different from the first position information and determine the target imaging position corresponding to the medical imaging device based on the internal human body image corresponding to the second position information, if the internal human body image corresponding to the first position information does not correspond to the target photography position.

In a seventh aspect, embodiments of the present disclosure provide an image display device, the device comprising:

a first blood vessel image display module, configured to obtain a first blood vessel image of an imaging object obtained by the angiography device based on a first horizon-of-view parameter, and display the first blood vessel image; a second horizon-of-view parameter determination module, configured to determine a second horizon-of-view parameter based on a received parameter adjustment instruction and the first horizon-of-view parameter; wherein the parameter adjustment instruction includes a scaling adjustment ratio; a second blood vessel image display module, configured to determine a second blood vessel image based on the second horizon-of-view parameter and simultaneously display the second blood vessel image and the first blood vessel image.

In an eighth aspect, embodiments of the present disclosure provide an angiography device, comprising an imaging assembly, at least one display device and a controller;

wherein the imaging assembly is configured to obtain a first blood vessel image based on a first horizon-of-view parameter; the display device is configured to display the first blood vessel image and a second blood vessel image; the controller includes one or more processors and a memory having one or more programs, which, when the one or more programs are executed by the one or more processors, causes the one or more processors to implement the image display method as described in any of the above covered.

In a ninth aspect, embodiments of the present disclosure further provide generating a volume reconstruction image, comprising:

a projection data acquisition module configured to obtain projection data at each scanning angle; a target volume coordinate system generation module configured to construct a target volume coordinate system based on an initial volume coordinate system and a desired reconstruction direction; a target volume reconstruction image generation module configured to reconstruct the projection data according to the desired reconstruction direction under the target volume coordinate system to generate a target volume reconstruction image.

In a tenth aspect, embodiments of the present disclosure also provide a system for generating a volume reconstruction image, comprising: a control device and an image acquisition device;

wherein the control device includes a memory, a processor and computer programs stored in the memory and runnable on the processor, the processor executing the computer programs implementing a method for generating a volume reconstruction image as described in any one of the first aspect; the image acquisition device is configured to scan a scanned object at each of the scanning angles to obtain projection data at each of the scanning angles.

In an eleventh aspect, embodiments of the present disclosure provide a device for a dynamic fluoroscopy of a C-shaped arm device, comprising at least one processor and at least one memory device, the memory device being used to store instructions that, when the at least one processor executes the instructions, implement a method for a dynamic fluoroscopy as described in any of the embodiments of the present disclosure.

In a twelfth aspect, embodiments of the present disclosure provide a C-shaped arm imaging system comprising a radiation source, a detector, a memory, and a display, the radiation source including a tube, a high voltage generator, and a high voltage control module; wherein the high voltage control module controls a reciprocal switching between a first energy and a second energy of the high voltage generator; the radiation source, driven by the high voltage generator, emits rays to a subject during a photography cycle, the detector obtains first fluoroscopic data of the subject irradiated by rays of the first energy and second fluoroscopic data of the subject irradiated by rays of the second energy; the memory stores the first fluoroscopic data and the second fluoroscopic data obtained in each of multiple successive photography cycles; the memory further stores an image processing unit, the image processing unit performing subtraction processing on the first fluoroscopic data and the second fluoroscopic data in each photography cycle in order to obtain an image of the subject in each photography cycle and thus dynamic images in the multiple photography cycles; a display is configured to display the dynamic images of the subject during the multiple photography cycles.

In a thirteenth aspect, embodiments of the present disclosure provide a medical imaging device, comprising, comprising:

one or more processors; a memory configured to store one or more programs; when the one or more programs are executed by the one or more processors such that the one or more processors implement an imaging positioning method for a medical imaging device as described in any of the above.

In a fourteenth aspect, embodiments of the present disclosure provide a computer-readable memory medium, comprising a set of instructions, wherein when executed by a processor, the method for a dynamic fluoroscopy described in any of the embodiments of the present disclosure is implemented.

In a fifteenth aspect, embodiments of the present disclosure provide a memory medium comprising computer-executable instructions, wherein the computer-executable instructions, when executed by a computer processor, are used to perform the imaging positioning method for a medical imaging device of any of the described above.

In a sixteenth aspect, embodiments of the present disclosure provide a memory medium comprising computer-executable instructions, wherein the computer-executable instructions, when executed by a computer processor, are used to perform the image display method of any of the described above.

In a seventeenth aspect, embodiments of the present disclosure provide a memory medium comprising computer-executable instructions, wherein the computer-executable instructions, when executed by a computer processor, are used to perform the method for generating a volume reconstruction image as described in any one of the former aspects.

## BRIEF DESCRIPTION OF THE DRAWINGS

In order to more clearly illustrate the technical solutions in the embodiments or prior art of the present disclosure, the following is a brief description of the drawings to be used in the description of the embodiments or prior art, it is obvious that the drawings in the following description are only embodiments of the present disclosure, and other drawings can be obtained according to the disclosed drawings without any creative work for those skilled in the art.

FIG. 1 is a schematic diagram illustrating an application scenario of a system for a dynamic fluoroscopy of a C-shaped arm device according to some embodiments of the present disclosure;
FIG. 2 is a flowchart illustrating an exemplary method for a dynamic fluoroscopy of a C-shaped arm device according to some embodiments of the present disclosure;
FIG. 3 is a flowchart illustrating an imaging positioning method for a medical imaging device according to some embodiments of the present disclosure;
FIG. 4 is a flowchart illustrating an imaging positioning method for a medical imaging device according to some embodiments of the present disclosure;
FIG. 5 is a schematic diagram illustrating an interactive interface according to some embodiments of the present disclosure;
FIG. 6 is a flowchart illustrating an imaging positioning method for a medical imaging device according to some embodiments of the present disclosure;
FIG. 7 is a schematic diagram illustrating an imaging scene of a virtual human body model according to some embodiments of the present disclosure;
FIG. 8 is a flowchart illustrating an image display method according to some embodiments of the present disclosure;
FIG. 9 is a flowchart illustrating an image display method according to some embodiments of the present disclosure;
FIG. 10 is a schematic flowchart illustrating a method for generating a volume reconstruction image according to some embodiments of the present disclosure;
FIG. 11 is a schematic diagram illustrating a definition of an initial volume coordinate system according to some embodiments of the present disclosure;
FIG. 12 is a schematic diagram illustrating a definition of a target volume coordinate system according to some embodiments of the present disclosure;
FIG. 13 is a schematic flowchart illustrating a method for generating a volume reconstruction image according to some embodiments of the present disclosure;
FIG. 14 is a diagram illustrating an exemplary module of a system for a dynamic fluoroscopy of a C-shaped arm device according to some embodiments of the present disclosure;
FIG. 15 is a schematic diagram illustrating an imaging positioning device for a medical imaging device according to some embodiments of the present disclosure;
FIG. 16 is a schematic diagram illustrating a structure of a medical imaging device according to some embodiments of the present disclosure;
FIG. 17 is a schematic diagram illustrating an image display device according to some embodiments of the present disclosure;
FIG. 18 is a schematic diagram illustrating a structure of an angiography device according to some embodiments of the present disclosure;
FIG. 19 is a schematic diagram illustrating a structure of a device for generating a volume reconstruction image according to some embodiments of the present disclosure;

FIG. 20 is a schematic diagram illustrating a structure of a system for generating a volume reconstruction image according to some embodiments of the present disclosure;

FIG. 21 is a schematic diagram illustrating a structure of a control device according to some embodiments of the present disclosure.

## DETAILED DESCRIPTION

The following is a clear and complete description of the technical solutions in the embodiments of the present disclosure in conjunction with the accompanying drawings in the embodiments of the present disclosure. Obviously, the described embodiments are only a part of the embodiments of the present disclosure, and not all of them. Based on the embodiments in the present disclosure, all other embodiments obtained by all those skilled in the art without creative labor fall within the scope of protection of the present disclosure.

The technical schemes of the present disclosure embodiments will be more clearly described below, and the accompanying drawings in the description of the embodiments will be briefly described below. Obviously, the drawings in the following description are merely some examples or embodiments of the present disclosure, and will be applied to other similar scenarios according to these accompanying drawings without paying creative labor. Unless obviously obtained from the context or the context illustrates otherwise, the same numeral in the drawings refers to the same structure or operation.

It should be understood that the "system", "device", "unit" and/or "module" used herein is a method for distinguishing different components, elements, units, parts or assemblies of different levels. However, if other words may achieve the same purpose, the words may be replaced by other expressions.

As shown in the present disclosure and claims, unless the context clearly prompts the exception, "a", "one", and/or "the" is not specifically singular form, and the plural form may be included. It will be further understood that the terms "comprise," "comprises," "comprising," "include," "includes," and/or "including," when used in present disclosure, specify the presence of stated features, integers, steps, operations, elements, and/or components, but do not preclude the presence or addition of one or more other features, integers, steps, operations, elements, components, and/or groups thereof.

While the present disclosure makes various references to certain modules or units in the system according to embodiments of the present disclosure, however, any number of different modules or units may be used and run on the client and/or server. The modules described are illustrative only and different modules may be used for different aspects of the systems and methods described.

Flow charts are used in the present disclosure to illustrate the operations performed by the system according to embodiments of the present disclosure. It should be understood that the preceding or following operations are not necessarily performed in precise order. Instead, the individual steps may be processed in reverse order or simultaneously. It is also possible to add other operations to these processes or to remove a step or steps of operations from these processes.

In the description of this present disclosure, it should be understood that the terms "center", "longitudinal", "transverse", "length", "width", "thickness", "upper", "lower", "front", "back", "left", "right", "vertical", "horizontal", "top", "bottom", "inside", "outside", "clockwise", "counterclockwise", "axial", "radial", "circumferential", or the like, indicate orientations or positional relationships based on those shown in the accompanying drawings and are intended only to facilitate and simplify the description of the present disclosure, do not indicate or imply that the referred device or part must have a particular orientation, be constructed and operate in a particular orientation, which cannot be interpreted as a limitation of the present disclosure.

In addition, the terms "first" and "second" are used for descriptive purposes only and are not to be construed as indicating or implying relative importance or implicitly specifying the number of technical features indicated. Thus, the features qualified with "first" and "second" may explicitly or implicitly include at least one such feature. In the description of this present disclosure, "multiple" or "a plurality of" means at least two, such as two, three, etc., unless otherwise expressly and specifically limited.

In the present disclosure, unless otherwise expressly specified and limited, the terms "mounted", "coupled", "connected", "fixed", etc. are to be understood in a broad sense, for example, which may be a fixed connection, a removable connection, or an integral part; a mechanical connection or an electrical connection; a direct connection or an indirect connection through an intermediate medium, a connection within two elements or an interaction between two elements. For a person of ordinary skill in the art, the specific meaning of the above terms in this present disclosure can be understood on a case-by-case basis.

In the present disclosure, unless otherwise expressly specified and limited, the first feature "above" or "below" the second feature may be direct contact between the first and second features, or indirect contact between the first and second features through an intermediate medium. Moreover, the first feature "above", "over" and "on" the second feature may be that the first feature is directly above or diagonally above the second feature, or simply indicate that the first feature is horizontally higher above the second feature. The first feature "under", "below", and "beneath" the second feature may be that the first feature is directly below or diagonally below the second feature, or simply indicate that the first feature is horizontally less than the second feature.

It should be noted that when an element is said to be "fixed to", "arranged on", or "set on" another element, it may be directly on the other element or there may be a centered element between the element and another element. When an element is considered to be "connected" to another element, it can be directly connected to another element or there may be both centered elements. The terms "vertical", "horizontal", "up", "down", "left", "right" and similar expressions used herein are for illustrative purposes only, and are not meant to be the only implementation.

Embodiment one

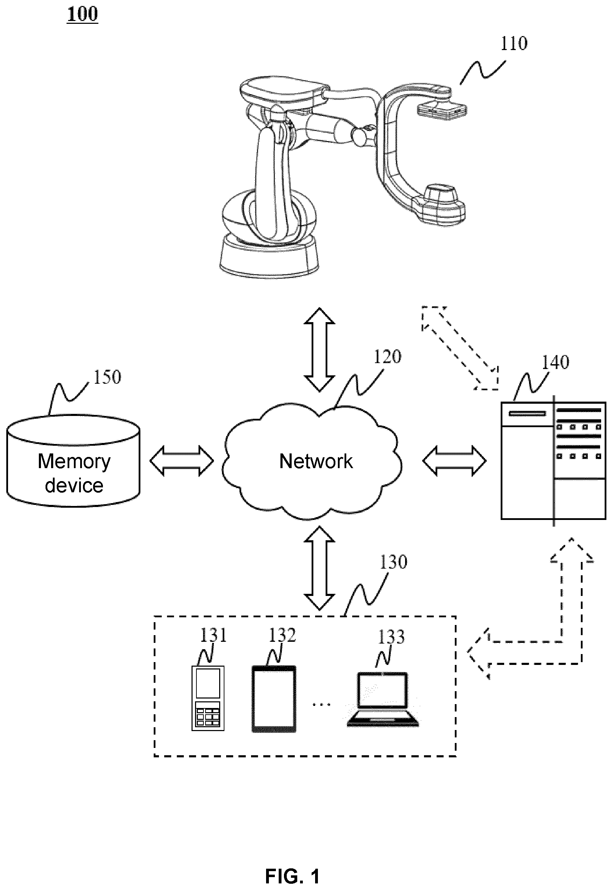

FIG. 1 is a schematic diagram illustrating an application scenario of a system for a dynamic fluoroscopy of a C-shaped arm device according to some embodiments of the present disclosure. The system 100 may include a fluoroscopy device 110, a network 120, at least one terminal 130, a processing device 140, and a memory device 150. Various components of the system 100 may be connected to each other via the network 120. For example, the fluoroscopy device 110 and the at least one terminal 130 may be connected or in communication with each other via the network 120.

The fluoroscopy device 110 may include a digital subtraction angiography (DSA), a digital radiography (DR) device, a computerized radiography (CR) device, a digital fluorography (DF) device, a computed tomography (CT) scanner, a magnetic resonance scanner, a mammography machine, a C-shaped arm device, or the like. In some embodiments, the fluoroscopy device 110 may include a frame, a detector, a detection region, a scanning couch, and a radiation source. The frame may be used to support the detector and the radiation source. The scanning couch may be used to hold a subject for scanning. The subject may include a patient, a mold, or other object being scanned. The radiation source may emit X-rays to the subject to irradiate the subject. The detector may be used to receive the X-rays. By photographing (i.e., irradiating) the subject for multiple photography cycles, the fluoroscopy device 110 may obtain fluoroscopic data in the multiple photography cycles to generate (or reconstruct) a dynamic image of the subject over the multiple photography cycles.

The network 120 may include any suitable network capable of facilitating the exchange of information and/or data of the system 100. In some embodiments, at least one component of the system 100 for a dynamic fluoroscopy (e.g., the fluoroscopy device 110, the processing device 140, the memory device 150, the at least one terminal 130) may exchange information and/or data with at least one other component of the system 100 via the network 120. For example, the processing device 140 may obtain an image of the subject from the fluoroscopy device 110 via the network 120. The network 120 may include a public network (e.g., the Internet), a private network (e.g., a local area network (LAN)), a wired network, a wireless network (e.g., an 802.11 network, a Wi-Fi network), a frame relay network, a virtual private network (VPN), a satellite network, a telephone network, a router, a hub, a switch, etc., or any combination thereof. For example, the network 120 may include a wired network, a wireless network, a fiber optic network, a telecommunications network, an Intranet, a wireless local area network (WLAN), a metropolitan area network (MAN), a public switched telephone network (PSTN), a Bluetooth™ network, a ZigBee™ network, a near field communication (NFC) network, or any combination thereof. In some embodiments, the network 120 may include at least one network access point. For example, the network 120 may include a wired and/or wireless network access point, such as a base station and/or an Internet exchange point, and at least one component of the system 100 may be connected to the network 120 via the access point to exchange data and/or information.

The at least one terminal 130 may be in communication and/or connection with the fluoroscopy device 110, the processing device 140, and/or the memory device 150. For example, first fluoroscopic data, second fluoroscopic data obtained by the processing device 140 may be stored in the memory device 150. In some embodiments, the at least one terminal 130 may include a mobile device 131, a tablet computer 132, a laptop computer 133, etc., or any combination thereof. For example, the mobile device 131 may include a mobile control handle, a personal digital assistant (PDA), a smartphone, or the like, or any combination thereof. In some embodiments, the at least one terminal 130 may include a display, and the display may be used to display information related to the dynamic fluoroscopy process (e.g., a dynamic image of the subject).

In some embodiments, the at least one terminal 130 may include an input device, an output device, or the like. The input device may optionally be a keyboard input, a touch screen (e.g., with haptic or tactile feedback) input, a voice input, an eye tracking input, a gesture tracking input, a brain monitoring system input, an image input, a video input, or any other similar input mechanism. Input information received via the input device may be transmitted to the processing device 140 via, for example, a bus for further processing. Other types of the input devices may include a cursor control device, for example, a mouse, a trackball, or a cursor arrow key. In some embodiments, an operator (e.g., a technician or a physician) may input, via the input device, an instruction that reflects a category of dynamic images selected by the user. The output device may include a display, a speaker, a printer, etc., or any combination thereof. The output device may be used to output dynamic images, etc., as determined by the processing device 140. In some embodiments, the at least one terminal 130 may be part of the processing device 140.

The processing device 140 may process data and/or information obtained from the fluoroscopy device 110, the memory device 150, the at least one terminal 130, or other components of the system 100. For example, the processing device 140 may obtain fluoroscopic data of the subject from the fluoroscopy device 110. In some embodiments, the processing device 140 may be a single server or group of servers. The group of servers may be centralized or distributed. In some embodiments, the processing device 140 may be local or remote. For example, the processing device 140 may access information and/or data from the fluoroscopy device 110, the memory device 150, and/or at least one terminal 130 via the network 120. As another example, the processing device 140 may connect directly to the fluoroscopy device 110, the at least one terminal 130, and/or the memory device 150 to access the information and/or data. In some embodiments, the processing device 140 may be implemented on a cloud platform. For example, the cloud platform may include a private cloud, a public cloud, a hybrid cloud, a community cloud, a distributed cloud, an inter-cloud, a multi-cloud, or any combination thereof.

The memory device 150 may store data, instructions, and/or any other information, for example, a historical photography protocol, etc. In some embodiments, the memory device 150 may store data obtained from the fluoroscopy device 110, the at least one terminal 130, and/or the processing device 140. In some embodiments, the memory device 150 may store data and/or instructions that the processing device 140 uses to perform or use to accomplish the exemplary methods described in the present disclosure. In some embodiments, the memory device 150 may include a mass memory, a removable memory, a volatile read-write memory, a read-only memory (ROM), etc., or any combination thereof. In some embodiments, the memory device 150 may be implemented on a cloud platform.

In some embodiments, the memory device 150 may be connected to the network 120 to communicate with at least one other component (e.g., the processing device 140, the at least one terminal 130) of the system 100. The at least one component of the system 100 may access data (e.g., fluoroscopic data) stored in the memory device 150 via the network 120. In some embodiments, the memory device 150 may be part of the processing device 140.

It should be noted that the above description is provided for illustrative purposes only and is not intended to limit the scope of the present disclosure. For those skilled in the art, a variety of variations and modifications may be made under the guidance of the contents of the present disclosure. The features, structures, methods, and other characteristics of the exemplary embodiments described in the present disclosure may be combined in various ways to obtain additional and/or alternative exemplary embodiments. For example, the memory device 150 may be a data memory device 150 that includes a cloud computing platform, such as a public cloud, private cloud, community, and hybrid cloud, etc. However, these variations and modifications will not depart from the scope of the present disclosure.

In some embodiments, the present disclosure also relates to an imaging system for a C-shaped arm. The imaging system for a C-shaped arm may include a radiation source, a detector, a memory, and a display, and the radiation source includes a tube, a high voltage generator, and a high voltage control module. In some embodiments, the high voltage control module controls a reciprocal switching between a first energy and a second energy of the high voltage generator; the radiation source, driven by the high voltage generator, emits rays to a subject during a photography cycle; the detector obtains first fluoroscopic data of the subject irradiated by rays of the first energy and second fluoroscopic data of the subject irradiated by rays of the second energy; the memory stores the first fluoroscopic data and the second fluoroscopic data obtained in each of multiple successive photography cycles; the memory further stores an image processing unit, the image processing unit performing subtraction processing on the first fluoroscopic data and the second fluoroscopic data in each photography cycle in order to obtain an image of the subject in each photography cycle and thus dynamic images in the multiple photography cycles; a display is configured to display the dynamic images of the subject during the multiple photography cycles.

In some embodiments, medical personnel need to frequently photograph the subject to understand the body condition of the subject. However, a picture only reflects the condition of the lesion and/or various tissues and organs of the subject at a certain moment in time, which often cannot meet the needs of the medical personnel. In particular, when performing clinical operations such as a surgery/diagnosis on a subject, the medical personnel need to understand the condition (e.g., movement) of the overall or local (e.g., bones, soft tissues, etc.) of the subject or the lesion of the subject in real time, and therefore need to utilize the fluoroscopy device 110 with a dynamic fluoroscopy function to take continuous images of the subject in one or more photography cycles to obtain a dynamic fluoroscopy image. However, in some embodiments, the fluoroscopy device with a fluoroscopy function is usually only able to irradiate the subject at one energy to obtain a fluoroscopy image, resulting in the inability to effectively use the photoelectric absorption effect and Compton scattering effect to obtain the images required by the healthcare provider, thereby reducing the efficiency of the medical personnel's diagnosis and clinical operation.

Therefore, some embodiments of the present disclosure provide a method for a dynamic fluoroscopy, which enables the radiation source to irradiate the subject in turn at different energies, so as to obtain the fluoroscopic data of the subject under different energy irradiation, and then obtains the dynamic fluoroscopic image of the subject required by the medical personnel based on the photoelectric absorption effect and Compton scattering effect, which effectively improves the clinical operation efficiency of the medical personnel and can better assist the medical personnel in diagnosis/therapy.

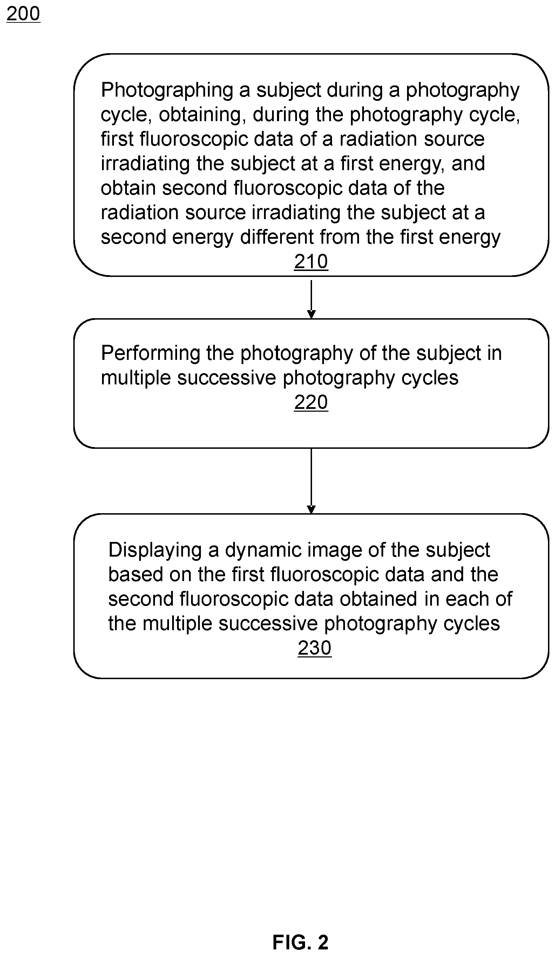

FIG. 2 is a flowchart illustrating an exemplary method for a dynamic fluoroscopy according to some embodiments of the present disclosure. Specifically, the method 200 for a dynamic fluoroscopy may be executed by the system 100 for a dynamic fluoroscopy (e.g., the processing device 140). For example, the method 200 for a dynamic fluoroscopy may be stored in a memory device (e.g., the memory device 150) in the form of a program or instructions that can be implemented when the system 100 for a dynamic fluoroscopy (e.g., the processing device 140) executes the program or instructions.

Step 210, photographing a subject during a photography cycle, obtaining, during the photography cycle, first fluoroscopic data of a radiation source irradiating the subject at a first energy as well as second fluoroscopic data of the radiation source irradiating the subject at a second energy different from the first energy. In some embodiments, step 210 may be performed by a photography module 310.

The subject may be a subject located at a position under the fluoroscopy device 110 that receives irradiation from the radiation source. In some embodiments, the subject may include an object for which a certain part (e.g., head, chest) is photographed, e.g., a patient, a person to be examined, etc. In some embodiments, the subject may also be a specific organ, tissue, part, etc.

The photography cycle can be understood as a time period during which the radiation source photographs the subject at the first energy and the second energy, respectively. In some embodiments, the photographing of the subject by the radiation source at the first energy and the photographing of the subject by the radiation source at the second energy may be consecutive, i.e., the photographing of the subject by the radiation source at the second energy is performed immediately after the photographing of the subject by the radiation source at the first energy. For ease of illustration, the radiation source photographs (i.e., irradiates) the subject at the first energy may also be referred to as a first energy photography and the radiation source photographs (i.e., irradiates) the subject at the second energy may also be referred to as a second energy photography. In some embodiments, the high voltage control module may control the high voltage generator to switch between the second energy and the first energy, so as to achieve the switching of the first energy photography and the second energy photography. In some embodiments, the first energy photography and the second energy photography may have a certain time interval between them. In some embodiments, the photography cycle may be 1/25 second, 1/50 second, etc.

In some embodiments, the fluoroscopy device 110 may include multiple radiation sources (e.g., a first radiation source and a second radiation source), with each radiation source emitting rays at different energies. For example, the first radiation source may emit a first radiation at the first energy and the second radiation source emits a second radiation at the second energy, the first energy may be lower or higher than the second energy. In some embodiments, the fluoroscopy device 110 may also have only one radiation source that can emit radiation at different energies under the control of the high voltage control module.

In some embodiments, an energy difference between the first energy and the second energy may be 5 KeV to 120 KeV. In some preferred embodiments, the energy difference between the first energy and the second energy may be 10 KeV to 90 KeV. In some embodiments, the energy difference between the first energy and the second energy may be 20 KeV to 100 KeV. In some embodiments, an available energy range of the first energy and an available energy range of the second energy may be partially overlapping or separated. For example, the available energy range of the first energy may be 60 KeV to 90 KeV, and the available energy range of the second energy is 100 KeV to 120 KeV. In some embodiments, the first energy may be 70 KeV and the second energy may be 120 KeV.

In some embodiments, the processing device 140 may obtain fluoroscopic data of the subject irradiated with rays of different energies (or referred to as a beam). For example, the processing device 140 may obtain first fluoroscopic data for the subject irradiated with rays of the first energy and second fluoroscopic data for the subject irradiated with rays of the second energy. In some embodiments, the fluoroscopic data may refer to data detected by the detector of the fluoroscopy device 110 after the rays have passed through the subject to be irradiated, and the memory of the fluoroscopy device 110 may store the first fluoroscopic data and the second fluoroscopic data obtained during each of multiple successive photography cycles. Further, an image processing unit in the processing device 140 or the memory may perform a processing (e.g., a subtraction processing) based on the fluoroscopic data to obtain images of the multiple photography cycles, and thus dynamic images of the multiple photography cycles. In some embodiments, the fluoroscopic data may also be understood as a reconstructed image based on the data detected by the detector. In some embodiments, the image may be reconstructed by using methods such as a semi-reconstruction method, a segmental reconstruction method, etc. In some embodiments, the processing device 140 may store the fluoroscopic data for subsequent processing of the fluoroscopic data to achieve the dynamic fluoroscopy.

It should be noted that the present disclosure does not limit the steps of photographing in one photography cycle. The system 100 for a dynamic fluoroscopy may first irradiate the subject by using the radiation source at the first energy to obtain the first fluoroscopic data, and then irradiate the subject by using the radiation source at the second energy to obtain the second fluoroscopic data. Alternatively, the system 100 for a dynamic fluoroscopy may first irradiate the subject by using the radiation source at the second energy, and then irradiate the subject by using the radiation source at the first energy. The specific photography steps can be selected according to the actual situation.

Step 220, photographing the subject in multiple successive photography cycles. In some embodiments, step 220 may be performed by the photography module 310.

In some embodiments, the multiple successive photography cycles may include a first photography cycle, a second photography cycle ...... an Nth photography cycle, with each photography cycle proceeding to the next photography cycle upon completion. In some embodiments, a photography cycle may start at a moment when the photography (e.g., the first energy photography) begins and end at a moment when the photography (e.g., the second energy photography) is completed. In this case, the irradiation of the subject by the radiation source at the second energy in a current photography cycle may proceed directly to the next photography cycle, i.e., to the step of irradiating the subject by the radiation source at the first energy. In some embodiments, for a photography cycle, a certain interval may also be included before the start of the first energy photography and/or after the completion of the second energy photography. In a specific embodiment, a photography cycle may start with a start moment of the first energy photography as the cycle start moment, and when the first energy photography is completed, a first time period may be the interval before the second energy photography; when the second energy photography is completed, a second time period may be the interval before ending the photography cycle and proceeding to the next photography cycle. In some embodiments, the first time period and the second time period may be equal. In one specific embodiment, a photography cycle is related to a frame rate, e.g., for the DSA device, 50 successive fluoroscopic images may be photographed in 1 second, where two successive frames are one photography cycle, i.e., 0.04 seconds.

Step 230, displaying a dynamic image of the subject based on the first fluoroscopic data and the second fluoroscopic data obtained in each of the multiple successive photography cycles. In some embodiments, step 230 may be performed by a display module 320.

In some embodiments, for each photography cycle, the display module 320 may generate an image of the subject (e.g., a target image) based on the first fluoroscopic data and the second fluoroscopic data obtained during that photography cycle. Multiple target images of the multiple successive photography cycles may form the dynamic image of the subject. The dynamic image of the subject may reflect changes in the subject (e.g., lesion, tissue, and/or organ, etc.) over the multiple successive photography cycles.

In some embodiments, the category of the dynamic image (or the target image) of the subject may include a soft tissue image, a skeletal image, and/or a combined image that includes at least a soft tissue and a skeleton. In some embodiments, the soft tissue image may refer to an image that shows only soft tissue portions. The skeletal image may refer to an image showing only a skeletal portion. The combined image may refer to an image showing at least a portion of the soft tissue and at least a portion of the skeleton. In some embodiments, the combined image may be formed by integrating the soft tissue image and the skeletal image according to a specific weighting. In some embodiments, the weighting of the soft tissue image and the skeletal image in the combined image may be determined based on the need of the medical personnel, for example, based on information input by the medical personnel via an input device.

In some embodiments, the processing device 140 (e.g., the display module 320) may determine the dynamic image of the subject and display it based on the first fluoroscopic data and the second fluoroscopic data obtained in each of the multiple successive photography cycles described above, using a dual-energy subtraction technique. Specifically, the intensity of the photoelectric absorption effect is positively correlated with the relative atomic mass of the irradiated material (e.g., the soft tissue, the skeletal portion of the subject, etc.) and is the primary way in which dense materials such as calcium, bone, iodine contrast agents, etc., attenuate X-ray photon energy. While the Compton scattering effect is independent of the relative atomic mass of the irradiated subject, it is a function of the electron density of the subject's body tissues and organs and occurs mainly in soft tissues. The dual-energy subtraction technique may determine the target image of the subject (e.g., the soft tissue image, the skeletal image, or the combined image) by taking advantage of the different energy attenuation patterns of X-ray photons by the skeleton and the soft tissue, and differences in the photoelectric absorption effects of substances of different atomic weights. These differences in attenuation and absorption are more evident in X-beams of different energies, while the effect of the energy of the X-beam on the intensity of the Compton scattering effect is almost negligible. The display module 320 may process the first fluoroscopic data and the second fluoroscopic data by using the dual-energy subtraction technique to selectively remove or partially remove attenuation information from the skeleton or the soft tissue, thereby obtaining the soft tissue image, the skeletal image, or the combined image and displaying it in a display (e.g., the terminal 130). In some embodiments, the image processing unit in the memory may also process the first fluoroscopic data and the second fluoroscopic data.

In some embodiments, the first fluoroscopic data may include a first projection image and the second fluoroscopic data may include a second projection image; the processing device 140 may determine a grayscale $Il(x,y)$ of each pixel point in the first projection image and a grayscale $Ih(x,y)$ of each pixel point in the second projection image. Based on the grayscale of each pixel point in the first projection image, the grayscale of each pixel point in the second projection image, and a subtraction parameter $\omega$, the processing device 140 may determine the grayscale of each pixel point in the target image based on equation (1). The subtraction parameter w may be set according to different parts of the subject.

$$Ides(x, y) = Il(x, y)/Ih(x, y) \ \omega \hspace{4cm} (1)$$

In other embodiments, the processing device 140 may also determine a first target image by overlapping the first projection image with the second projection image; and then determine a second target image by inverse coloring the first target image. The first target image and the second target image may be dynamic images of different categories, for example, the first target image may be a skeletal image and the second target image may be a soft tissue image.

In some embodiments, the processing device 140 may obtain an instruction input by a user (e.g., a medical practitioner, a technician), which may reflect the user's selection of the category of dynamic images to be displayed, and then determine and display the dynamic images of the subject based on the instruction. The medical personnel may select the category of a dynamic image generated by the fluoroscopy device 110 according to the actual needs, for example, when operating/diagnosing a skeletal site, the medical personnel may choose to generate and display a skeletal image; when operating/diagnosing a soft tissue site, the medical personnel may choose to generate and display a soft tissue image; when both a skeletal site and a soft tissue site need to be viewed, the medical personnel may choose to generate and display a combined image. In some embodiments, the instruction may include a selection instruction, a voice instruction, or a text instruction, etc. The voice instruction may be voice information input by the medical personnel via the input device, e.g., "display a skeletal image". The text instruction may be textual information input by the medical personnel through the input device, for example, "display a soft tissue image" on the input device. The selection instruction may be an instruction or selection item that is displayed on the interface of the input device for selection by the medical personnel, e.g., selection item 1: "display a skeletal image", selection item 2: "display a soft tissue image", selection item 3: "display a combined image".

Embodiment two

When a medical device is used to diagnose or treat a patient's tissue site, it is important to ensure that the medical device can be positioned to a target tissue site so that the pathological characteristics of the target tissue site can be accurately observed or the treatment effect can be improved.

To achieve the above, the widely adopted approach of prior art is to use X-rays to pass through the human body, different tissue parts have different densities and thus absorb different degrees of X-rays, and according to the nature of differential absorption tissue parts with different densities can be distinguished to reflect the tissue information inside the human body. However, the damage caused by ionizing radiation to the human body is very extensive and unpredictable.

In the multiple embodiments presently disclosed, the medical imaging device is described as an example of a DSA device, and it can be understood by those skilled in the art that other medical imaging devices are also available in the following embodiments. Typically, the DSA device includes a C-shaped arm, a radiation source, and a detector, wherein the radiation source and detector are mounted at ends of the C-shaped arm, and the C-shaped arm may be supported by a movable frame, which may be suspended or floor-mounted. The frame may also be a robot type frame. During imaging, the imaging object is lying on a hospital bed, and located between the radiation source and the detector. The DSA device fluoroscopes the imaging object to obtain multiple frames of internal images of the human body to assist a physician in operations such as surgery and guidewire insertion.

The present disclosure is described in further detail below in connection with the accompanying drawings and embodiments. It can be understood that the specific embodiments described herein are for the purpose of explaining the present disclosure only, and not for the purpose of limiting the present disclosure. It should also be noted that, for ease of description, the accompanying drawings show only those portions of the structure that are relevant to the present disclosure and not all of it.

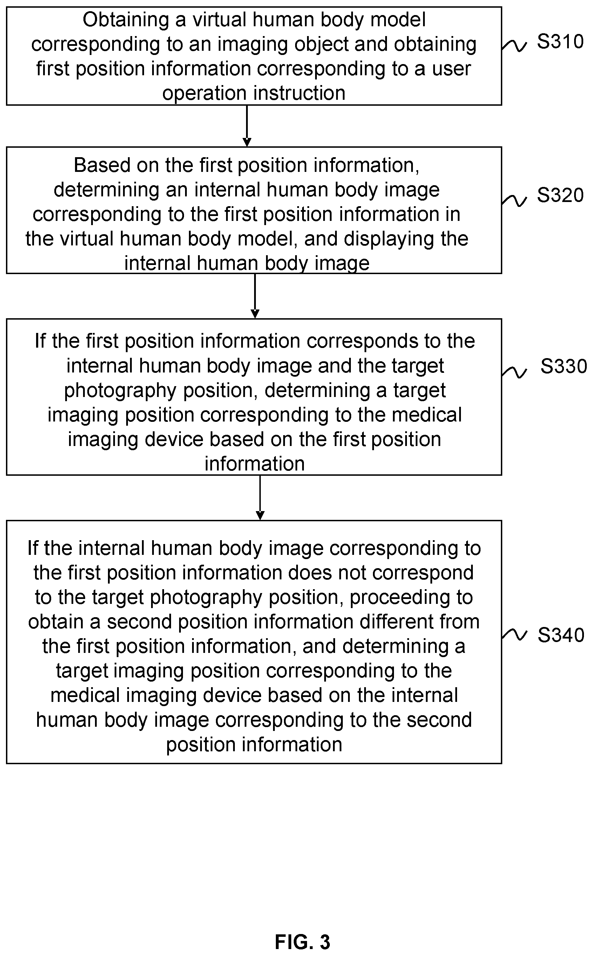

FIG. 3 is a flowchart illustrating an imaging positioning method for a medical imaging device according to some embodiments of the present disclosure. This embodiment may be applicable to the case of positioning a target site, which may be performed by the imaging positioning method, and the device may be implemented in software and/or hardware.

The specific steps include as follows:

Step 310, obtaining a virtual human body model corresponding to an imaging object and obtaining first position information corresponding to a user operation instruction.

In one embodiment, optionally, the obtaining a virtual human body model corresponding to an imaging object includes: according to obtained height data corresponding to the imaging object, selecting a virtual human body model corresponding to the height data; wherein the virtual human body model includes a human body size model and an internal human body model.

Specifically, the virtual human body model consists of two parts of the model, one part is a human body size model related to the height data and the other part is an internal human body model corresponding to the human body size model. The virtual human body model may be a two-dimensional model or a three-dimensional model.

The obtaining of the height data corresponding to the imaging object may include either receiving the height data corresponding to the imaging object inputted by the user or obtaining historical medical images of the imaging object and determining the height data based on image dimensions between key points in the historical medical images. Exemplarily, the key points may include a boundary point or an articulation point.

In one embodiment, optionally, the height data is matched with at least one stored height interval data, and a virtual human body model corresponding to successfully matched height interval data is used as the virtual human body model corresponding to the height data. Exemplarily, the height interval data may be 1.0-1.1 m, 1.1-1.2 m, 1.2-1.3 m, 1.3-1.4 m, 1.4-1.5 m, 1.5-1.6 m, 1.6-1.7 m, 1.7-1.8 m, and 1.9-2.0 m, etc., each height interval data corresponding to a human body size model. As such, a mean height corresponding to the height interval data is used as a height of the human body size model corresponding to the height interval data. For example, the height of the human body size model corresponding to the height interval data of 1.0-1.1 m may be 1.05 m.

It can be understood that a count of this height interval data may be smaller or larger, and accordingly, a count of reference human body models may be larger or smaller. Of course, it is also possible to add a height interval data of 2.0-2.1 m to the above height interval data or to delete a height interval data of 1.0-1.1 m.

In one embodiment, optionally, the internal human body model includes at least one of a blood vessel model, an organ model, a skeletal model, and a muscle model.

In one embodiment, optionally, the human body size model and the internal human body model are stored correspondingly based on the height interval data. Exemplarily, for height interval data of 1.0-1.1m, 1.1-1.2m, 1.2-1.3m, 1.3-1.4m, 1.4-1.5m, 1.5-1.6m, 1.6-1.7m, 1.7-1.8m, and 1.9-2.0m, each height interval data corresponds to an internal human body model.

In one embodiment, optionally, when height data does not match height interval data, a virtual human body model matched with the height data is determined based on the virtual human body model corresponding to each of the two height interval data adjacent to the height data. For example, if the height data is 1.45 m, but the stored height interval data are 1.0-1.1 m, 1.1-1.2 m, 1.2-1.3 m, 1.3-1.4 m, 1.5-1.6 m, 1.6-1.7 m, 1.7-1.8 m and 1.9-2.0 m. Then a virtual human body model corresponding to the 1.4-1.5m height interval data is estimated based on a virtual human body model corresponding to 1.3-1.4m and a virtual human body model corresponding to 1.5-1.6m, and the estimated virtual human body model is used as the virtual human body model matched with the height data. Exemplarily, the estimation method may be a method such as difference or average, and the specific estimation method is not limited here. The advantage of such a setup is to improve the match between the obtained virtual human body model and the imaging object as much as possible, and thus reduce the error between the subsequent images of the internal human body and the real tissue images.

In another embodiment, optionally, the first position information includes a first model information corresponding to the virtual human body model or a first device information corresponding to the medical imaging device, wherein there is an association relationship between the first model information and the first device information. Specifically, based on the association relationship between the two, when the first model information changes, the first device information also changes, conversely, when the first device information changes, the first model information also changes.

In yet another embodiment, optionally, the association relationship includes a position association relationship. The method further includes: converting a relative position relationship between the medical imaging device and the imaging object into a position association relationship between the medical imaging device and the virtual human body model. The position association relationship is used to characterize a relationship between position parameters of the first model information and the first device information.

Exemplarily, the medical imaging device should establish the relative position relationship between the two by matching a preset datum point or preset datum line to the imaging object. The preset datum point or the preset datum line may be a preset datum point or datum line on a treatment bed. Accordingly, the preset datum line may be a datum line aligned with the head vertex of the imaging object on the treatment bed.

Specifically, the first model information includes a model position parameter corresponding to the virtual human body model, and the first device information includes a device position parameter corresponding to the medical imaging device. Exemplarily, the model position parameter may be a center point position and a model angle corresponding to the internal human body image, and the device position parameter may be a center position of the radiation source and an angle of the axis between the radiation source and the detector with respect to the treatment bed.

In one embodiment, optionally, the association relationship further includes a horizon-of-view association relationship, the method further includes: obtaining a horizon-of-view association relationship between the first model information and the first device information; wherein the horizon-of-view association relationship is used to characterize a relationship between horizon-of-view parameters of the first model information and the first device information.

Specifically, the first model information includes a model horizon-of-view parameter corresponding to the virtual human body model, and the first device information includes a device horizon-of-view parameter corresponding to the medical imaging device. The model horizon-of-view parameter may be an image dimension corresponding to the internal human body image, and the device horizon-of-view parameter may be at least one of a magnification, a source image distance, and a source object distance. Accordingly, a mapping list may be pre-established to describe the horizon-of-view association relationship between the first model information and the first device information.

S 320, based on the first position information, determining an internal human body image corresponding to the first position information in the virtual human body model, and displaying the internal human body image.

Exemplarily, the internal human body image is a virtual image of the virtual human body model corresponding to the first position information. Specifically, when the virtual human body model is a three-dimensional model, the internal human body image may be a three-dimensional image or a two-dimensional projection image corresponding to angle information in the first position information.

In one embodiment, optionally, the internal human body image includes a model image corresponding to at least one internal human body model of the virtual human body model, wherein the internal human body model includes at least one of a blood vessel model, an organ model, a skeletal model, and a muscle model. In one embodiment, a user may select the internal human body model corresponding to the internal human body image on an interactive interface. If the user selects the blood vessel model before imaging the blood vessels, the internal human body image displays only the model image of the blood vessel model. In another embodiment, the user may select a transparency of the internal human body model on the interactive interface. If the transparency of the blood vessel model is 0% and the transparency of the other models is 100%, then the human internal image also shows only the model image of the blood vessel model. The advantage of such a setting is to minimize the visual interference to the user caused by models other than the target model, thus improving the accuracy of the subsequent positioning of the internal human body image.

S 330, if the internal human body image corresponding to the first position information corresponds to the target photography position, determining a target imaging position corresponding to the medical imaging device based on the first position information.

Accordingly, the target photography position may be a preset photography position by the user according to an imaging plan before imaging positioning. Further, the target photography position may be a body position such as chest, head and abdomen, or an organ position such as stomach, small intestine, esophagus and throat. The specific setting of the target photography position is not limited here. If the internal human body image contains an image corresponding to the target photography position, the internal human body image is considered to correspond to the target photography position.

S 340, if the internal human body image corresponding to the first position information does not correspond to the target photography position, proceeding to obtain second position information different from the first position information, and determining a target imaging position corresponding to the medical imaging device based on an internal human body image corresponding to the second position information.

Specifically, when the first position information is the first model information, the second position information is the second model information; when the first position information is the first device information, the second position information is the second device information. The technical solution of this embodiment is that by determining the internal human body image corresponding to the first position information input by the user based on the virtual human body model, and by determining whether the internal human body image corresponds to the target photography position, the corresponding target imaging position of the medical imaging device is obtained, which solves the excessive radiation to the human body during the imaging operation and enables the user to set any parameters in the process of imaging and positioning without causing any damage to the human body, thus also ensuring the positioning accuracy.

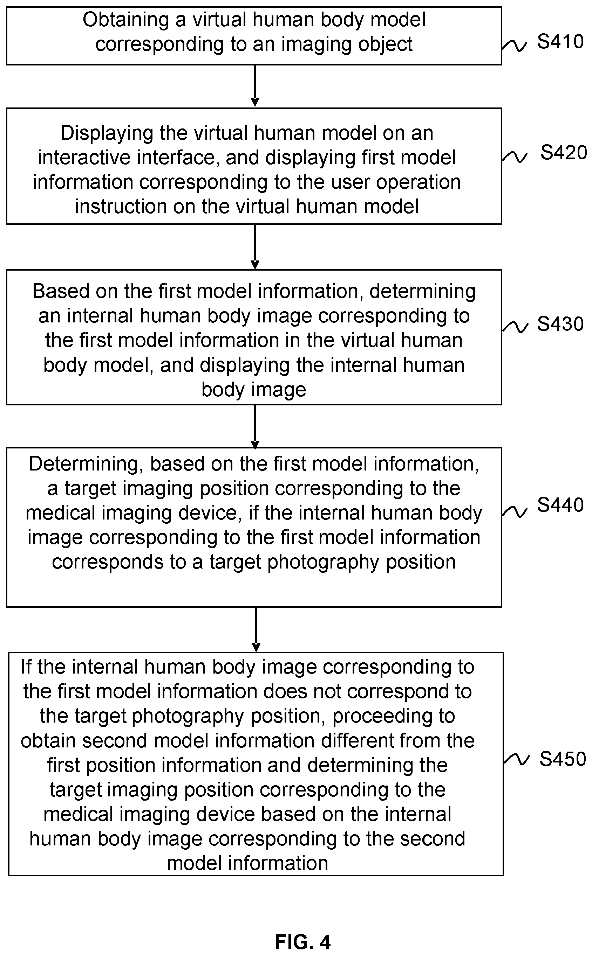

FIG. 4 is a flowchart illustrating an imaging positioning method for a medical imaging device according to some embodiments of the present disclosure, the technical solution of this embodiment is detailed description based on the above embodiment. Optionally, the obtaining the first position information corresponding to the user operation instruction includes: displaying the virtual human body model on an interactive interface when the first position information is first model information, and displaying the first model information corresponding to the user operation instruction on the virtual human body model.

The specific implementation steps of this embodiment include:

S 410, obtaining a virtual human body model corresponding to an imaging object.
S 420, displaying the virtual human body model on an interactive interface, and displaying first model information corresponding to the user operation instruction on the virtual human body model.

In one embodiment, optionally, the first model information includes a graphical marker.

Exemplarily, the shape of the graphical marker may be a square, a circle, a diamond, or any shape, and the specific shape of the graphical marker is not limited here. In one embodiment, optionally, the graphical marker performs at least one of selecting, moving, zooming in, and zooming out based on the user operation instruction. Further, the graphical marker corresponding to a selection operation instruction is displayed when the selection operation instruction is received from the user. Particularly, when the virtual human body model is a three-dimensional model, the graphical marker may be moved not only in the XOY plane in which the virtual human body model is located, but also in the XOZ plane and/or the YOZ plane in which the virtual human body model is located, and in addition, the movement includes a translation and a rotation. Furthermore, by moving the graphical marker, a model image on any level and angle of the virtual human body model can be selected.

S 430, based on the first model information, determining an internal human body image corresponding to the first model information in the virtual human body model, and displaying the internal human body image.

In one embodiment, when the first model information is a graphical marker, the internal human body image corresponding to the graphical marker is determined based on a dimension of the graphical marker and a position of the graphical marker relative to the virtual human body model. Specifically, the position of the graphical marker relative to the virtual human body model includes an angle and a position coordinate of the graphical marker relative to the virtual human body model.

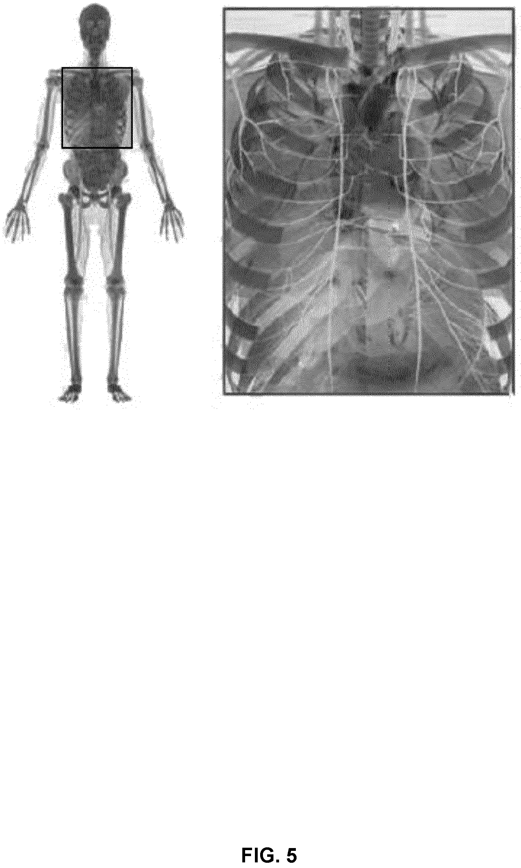

FIG. 5 is a schematic diagram illustrating an interactive interface according to some embodiments of the present disclosure. As shown in FIG. 5, the left image contains the virtual human body model and the graphical marker (black box) on the virtual human body model, and the right image represents the internal human body image corresponding to the graphical marker in the virtual human body model.

S 440, determining, based on the first model information, a target imaging position corresponding to the medical imaging device, if the internal human body image corresponding to the first model information corresponds to a target photography position.

S 450, if the internal human body image corresponding to the first model information does not correspond to the target photography position, proceeding to obtain second model information different from the first model information and determining the target imaging position corresponding to the medical imaging device based on the internal human body image corresponding to the second model information.

In one embodiment, optionally, before proceeding to obtain second position information different from the first position information, the method further includes: when the first position information is first model information, determining a first imaging position corresponding to the medical imaging device based on the first model information and the association relationship, and controlling the medical imaging device to move to the first imaging position. The technical effect achieved by this embodiment is that during the imaging process of the medical imaging device, the user views the internal human body image by inputting at least one first model information, and during the viewing process, an imaging component corresponding to the medical imaging device moves with the change of the first model information. When the second model information corresponds to the internal human body image and the target photography position, a current imaging position of the imaging component of the medical imaging device is the target imaging position.

In another embodiment, optionally, the determining a target imaging position corresponding to the medical imaging device based on the first position information includes: when the first position information is first model information, determining the target imaging position corresponding to the medical imaging device based on the first model information and the association relationship, and controlling the medical imaging device to move to the target imaging position. The technical effect achieved by this embodiment is that during the imaging process of the medical imaging device, the user views the internal human body image by inputting at least one type of first model information, and during the viewing process, an imaging component of the medical imaging device may not move with the change of the first model information. When a positioning instruction is received from the user input, the medical imaging device is controlled to move to the target imaging position from an initial position based on the target device information.

Optionally, in another embodiment, after controlling the medical imaging device to move to the target imaging location based on the target device information, the method further includes: controlling the imaging component in the medical imaging device to perform an imaging operation based on a horizon-of-view parameter; wherein the horizon-of-view parameter includes at least one of a source image distance, a source object distance, and a magnification. Specifically, the source image distance represents a distance between the radiation source and the detector, the source object distance represents a distance between the radiation source and the imaging object, and the magnification represents a magnification of the imaging image.

The technical solution of this embodiment, by displaying the virtual human body model and the graphical marker on the interactive interface, solves the problem of complicated direct input of the first model information by the user, who can intuitively observe the virtual human body model and preview the internal structure of the human body in any relative position by changing the position of the graphical marker in relation to the virtual human body model without the need for perspective view of the actual human body.

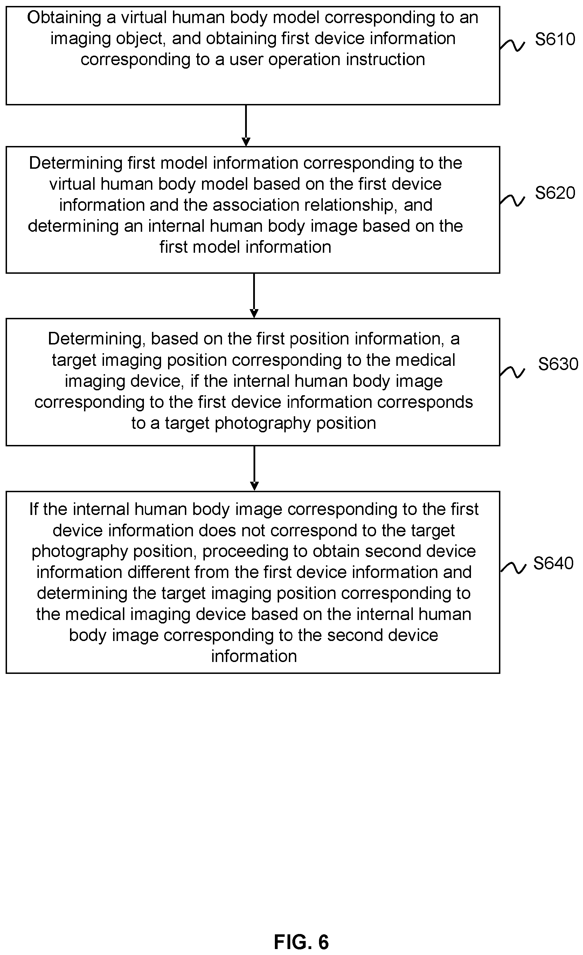

FIG. 6 is a flowchart illustrating an imaging positioning method for a medical imaging device according to some embodiments of the present disclosure. The technical solution of the embodiment is a further detailed description on the basis of the above embodiment. Optionally, the determining an internal human body image corresponding to the first position information in the virtual human body model based on the first position information includes: when the first position information is first device information, determining the first model information corresponding to the virtual human body model based on the first device information and the association relationship, and determining the internal human body image based on the first model information.

The specific implementation steps of this embodiment include:

S 610, obtaining a virtual human body model corresponding to an imaging object, and obtaining first device information corresponding to a user operation instruction.

The user operation instruction may be a parameter-input operation instruction that receives the first device information input by the user. In one example, the device parameter information of the medical imaging device input by the user may be used as the first device information; in another example, because a system coordinate of the medical imaging device is previously calibrated, the coordinates of the various components of the medical imaging device at each position may be updated in real time and known by the system. Thus, an operator, such as a doctor, moves the medical imaging device from a first position to a second position, and then the system is able to know the system coordinates of the various components of the medical imaging device, whether in the first position or the second position.

S 620, determining first model information corresponding to the virtual human body model based on the first device information and the association relationship, and determining an internal human body image based on the first model information.

For instance, the first device information input by the user is converted into the first model information corresponding to the virtual human body model according to the association relationship, and the internal human body image corresponding to the first model information is determined.

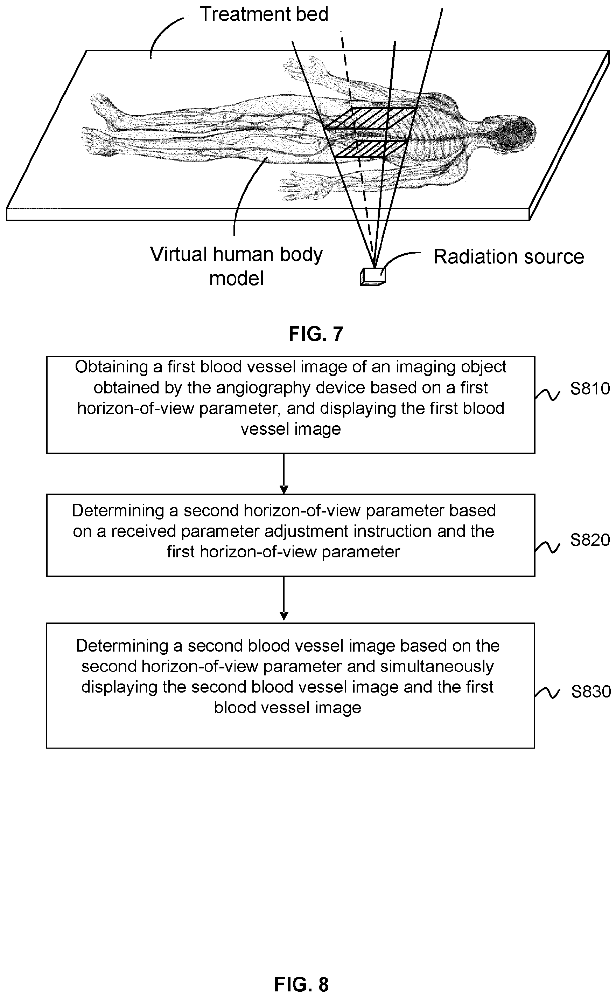

In another instance, the association relationship includes a matching relationship between a horizon-of-view parameter, such as a source image distance, in the first device information and an image depth in the first model information. The source image distance represents a distance between the radiation source and the detector. FIG. 7 is a schematic diagram illustrating an imaging scene of a virtual human body model according to some embodiments of the present disclosure. The three solid lines and one dashed line from the radiation source indicate a beam emitted from the radiation source, i.e., a conical beam emitted from the radiation source passes through the virtual human body model. FIG. 7 illustrates a horizon-of-view range corresponding to different depth levels of the virtual human body model during the propagation of the beam. It can be visualized from FIG. 7 that the horizon-of-view range corresponding to the different depth levels in the propagation path of the beam varies.

In one embodiment, the medical imaging device includes a digital X-ray photography device, a C-shaped arm X-ray device, a mammography machine, a computed tomography photography device, a magnetic resonance device, a positron emission tomography (PET) device, a positron emission tomography and computed tomography (PET-CT) device, a positron emission tomography and magnetic resonance imaging (PET-MR) device, or a radiotherapy (RT) device.

S 630, determining, based on the first position information, a target imaging position corresponding to the medical imaging device if the internal human body image corresponding to the first device information corresponds to a target photography position.

In another embodiment, the imaging position in the first device information is used as a target imaging position corresponding to the medical imaging device.

Optionally, based on the above embodiment, the method further includes: displaying the virtual human body model on an interactive interface and displaying a graphical marker on the virtual human body model based on the first device information. Specifically, the first model information is determined based on the first device information and second model information, and the graphical marker is displayed based on the first model information. The advantage of such a setting is that after the user inputs the first device information, the user can also continue to input the first model information by performing operations such as selecting, moving, zooming in or zooming out on the graphical marker. The first device information and the second model information are input alternately to achieve the determination of the internal human body image from different dimensions and further improve the positioning accuracy.

S 640, if the internal human body image corresponding to the first device information does not correspond to the target photography position, proceeding to obtain second device information different from the first device information and determining the target imaging position corresponding to the medical imaging device based on the internal human body image corresponding to the second device information.

The technical solution of this embodiment can be quickly located by receiving the first device information input by the user and displaying the internal human body image based on the association relationship between the first device information and the first model information.

In one embodiment, the contents disclosed in embodiment one above may be applied in embodiment two. For example, after determining the target imaging position of the imaging object (wherein the imaging object is the subject in embodiment one) based on what is disclosed in embodiment two, the imaging object is photographed during a photographical cycle, during which first fluoroscopic data of the radiation source irradiating the imaging object at a first energy is obtained, and second fluoroscopic data of the radiation source irradiating the imaging object at a second energy different from the first energy is obtained. The target imaging position of the imaging object is photographed in multiple successive photography cycles. Afterwards, a dynamic image of the imaging object is displayed based on the first fluoroscopic data and the second fluoroscopic data obtained in each of the multiple successive photography cycles.

Embodiment three

Angiography is an auxiliary examination technique that uses the principle that X-rays cannot penetrate the contrast agent to observe the lesions of blood vessels, and is commonly used in clinical diagnosis and treatment of various diseases. At the same time, angiography is a minimally invasive technique, which requires the insertion of a catheter into the blood vessel to be tested so that the contrast agent can be injected into the blood vessel to be tested, and then the imaging device is used to image the blood vessel to be tested.

When viewing an imaging result, the physician needs to constantly zoom in or out to obtain comprehensive blood vessel information, thus constantly switching the images at different views on the display device.

Based on the above existing technical solutions, when the physician performs angiography, if he/she needs to compare different horizon-of-view images, he/she needs to switch the images constantly. Since the user needs to constantly switch the blood vessel images on the display device, the imaging object has to be rescanned each time the switch is made, which in turn reduces the diagnostic efficiency of angiography, and at the same time repeated switching also increases the risk of error in the diagnostic results of angiography.

FIG. 8 is a flowchart illustrating an image display method according to some embodiments of the present disclosure, this embodiment may be applicable to the use of an angiography device for imaging and displaying an imaging result, the method may be performed by an image display device, which may be implemented in software and/or hardware, the device may be configured in the angiography device. Specifically, the steps include as follows:

S 810, obtaining a first blood vessel image of an imaging object obtained by the angiography device based on a first horizon-of-view parameter, and displaying the first blood vessel image.

The angiography technique is a technique to visualize the blood vessels of X-ray sequence images by injecting a contrast agent into the blood vessels. Since X-rays cannot penetrate the contrast agent, the angiography uses X-rays to scan and image the tested site, and the contrast agent makes the blood vessels in the tested site have material density differences with other tissues, so that the images obtained from the scan can accurately reflect the site and extent of the blood vessel lesions.

Exemplarily, the first horizon-of-view parameter includes, but is not limited to, at least one of a first horizon-of-view range, a first magnification, a first horizon-of-view center position, and a first imaging mode. In the case of an imaging object being the abdomen, specifically, the first horizon-of-view range may be used to describe a region corresponding to the imaging object in the first blood vessel image. Exemplarily, assuming that the overall region size corresponding to the abdomen is 100*100, the first horizon-of-view range may be 100*100 or 30*50, and when the first horizon-of-view range is 30*50, the first horizon-of-view range includes a portion of the horizon-of-view range of the abdomen. Specifically, the first magnification may be used to describe a magnification of the first blood vessel image to the imaging object, similar to magnifying the blood vessel on the image by 100 times, etc. The first magnification may be either a relative magnification relative to a standard magnification or an actual magnification relative to the image to the blood vessel. If the first magnification is a relative magnification relative to the standard magnification, when the first magnification is greater than the standard magnification, the image corresponding to the first blood vessel image relative to the standard magnification is the image that is enlarged, and when the first magnification is less than the standard magnification, the image corresponding to the first blood vessel image relative to the standard magnification is the image that is reduced.

For example, the first horizon-of-view center position may be used to describe an image center position of the first blood vessel image, and from the perspective of the angiographical device, the first horizon-of-view center position may be used to describe a position on the detector corresponding to a beam center position of the radiation source. Specifically, the first imaging mode includes a fluoroscopic mode or an exposure mode. The fluoroscopic mode is an imaging mode with a high radiation dose and a low image resolution, and the exposure mode is an imaging mode with a low radiation dose and a high image resolution. Specifically, in a practical application scenario, the user may switch the imaging mode by using the Zoom function on the angiography device.

In one embodiment, the displaying the first blood vessel image includes: displaying the first blood vessel image in a preset display region of the display device, or displaying the first blood vessel image on an entire display interface of the display device.

S 820, determining a second horizon-of-view parameter based on a received parameter adjustment instruction and the first horizon-of-view parameter.

In this embodiment, the parameter adjustment instruction includes a scaling adjustment ratio. Specifically, when the scaling adjustment ratio is greater than one, a second magnification of the second horizon-of-view parameter is greater than the first magnification or a second horizon-of-view range is less than the first horizon-of-view range. When the scaling adjustment ratio is less than one, the second magnification of the second horizon-of-view parameter is smaller than the first magnification or the second horizon-of-view range is larger than the first horizon-of-view range. From the perspective of the blood vessel image, when the scaling adjustment ratio is greater than one, a magnification operation is performed on the first blood vessel image. When the scaling adjustment ratio is less than one, a reduction operation is performed on the first blood vessel image.

When the parameter adjustment instruction includes only the scaling adjustment ratio, if the scaling adjustment ratio is 1, the first horizon-of-view parameter is the second horizon-of-view parameter at this time, and the second blood vessel image subsequently obtained is identical to the first blood vessel image. In one embodiment, optionally, after determining the second horizon-of-view parameter based on the received parameter adjustment instruction and the first horizon-of-view parameter further includes: generating prompt information if the first horizon-of-view parameter is the same as the second horizon-of-view parameter. Specifically, the prompt information may be used to indicate to the user that a blood vessel image corresponding to the parameter adjustment instruction already exists. Further, the prompt information may be used to indicate to the user the display position of the blood vessel image corresponding to the current horizon-of-view adjustment parameter. Exemplarily, the prompt manner may be at least one of a text prompt, a voice prompt, and a prompt light. A corresponding prompt light may be set for at least one display device, and the prompt light flashes if the currently displayed blood vessel image on the display device corresponds to the current parameter adjustment instruction. The advantage of such a setting is to avoid displaying blood vessel images with the same horizon-of-view parameters, which causes waste of display positions and reduces the utilization of the display device.

In another embodiment, the determining the second horizon-of-view parameter based on the received parameter adjustment instruction and the first horizon-of-view parameter includes: determining a second magnification of the second horizon-of-view parameter based on the scaling adjustment ratio and the first magnification of the first horizon-of-view parameter. Exemplarily, assuming that the first magnification of the first horizon-of-view parameter is 2 times relative to a preset standard magnification and the scaling adjustment ratio is 3 times, the second magnification is 6 times.

In one embodiment, optionally, the determining the second horizon-of-view parameter based on the received parameter adjustment instruction and the first horizon-of-view parameter includes: determining a second horizon-of-view range of the second horizon-of-view parameter based on the scaling adjustment ratio and the first horizon-of-view range of the first horizon-of-view parameter. Exemplarily, assuming that the first horizon-of-view range is 10*10 and the scaling adjustment ratio is 2 times, the second horizon-of-view range is 5*5.

Based on the above embodiment, when the parameter adjustment instruction contains only the scaling adjustment ratio, the second magnification and the second horizon-of-view range are calculated based on the first magnification, the first horizon-of-view range and the scaling adjustment ratio of the first horizon-of-view parameter. Exemplarily, a second horizon-of-view center position may default to the first horizon-of-view center position in the first horizon-of-view parameter, and a second imaging mode defaults to the first imaging mode in the first horizon-of-view parameter.

Based on the above embodiment, optionally, the parameter adjustment instruction also includes at least one of a horizon-of-view adjustment range, a horizon-of-view adjustment center position, and an imaging adjustment mode. Specifically, the user may customize the second horizon-of-view range, the second horizon-of-view center position and the second imaging mode in the second horizon-of-view parameter. Specifically, the horizon-of-view adjustment range, the horizon-of-view adjustment center position and the imaging adjustment mode in the parameter adjustment instruction may be used as the second horizon-of-view range, the second horizon-of-view center position and the second imaging mode, respectively. The parameter adjustment instruction may be generated based on a parameter value input by the user or by obtaining a selection operation generated by the user based on the displayed first blood vessel image input. Exemplarily, the user may implement an input of the scaling adjustment ratio by using the Zoom function on the angiography device. The user may also input an image selection frame based on the first blood vessel image and generate a horizon-of-view adjustment range based on the size of the input image selection frame.

S 830, determining a second blood vessel image based on the second horizon-of-view parameter and simultaneously displaying the second blood vessel image and the first blood vessel image.

In one embodiment, optionally, the determining a second blood vessel image based on the second horizon-of-view parameter includes: obtaining the second blood vessel image obtained by the angiographical device based on the second horizon-of-view parameter. Specifically, the second blood vessel image may be obtained by controlling the angiography device based on the second horizon-of-view parameter.

It should be noted that the embodiment of the present disclosure is exemplarily explained and illustrated by simultaneous display of two blood vessel images. In practice, more blood vessel images corresponding to different horizon-of-view parameters can be obtained according to the image display method provided by the embodiment of the present disclosure, and the respective blood vessel images are displayed simultaneously. The number of times is not limited to the number of simultaneously displayed blood vessel images.

The technical solution of this embodiment, by determining a second blood vessel image based on the received parameter adjustment instruction while displaying the first blood vessel image and displaying the second blood vessel image simultaneously with the first blood vessel image, the problem of repeatedly switching the scanned images of different horizons of view can be solved, which reduces the number of repeated imaging and the amount of radiation, improves the service life of the angiographical device, which in turn also improves the diagnostic efficiency of angiography and reduces the error of the diagnostic results of angiography caused by repeated switching.

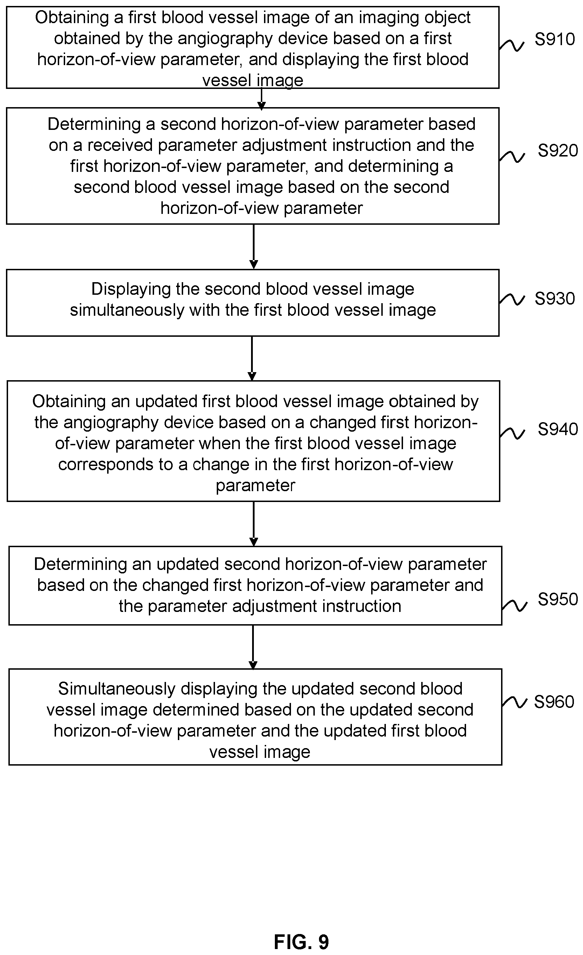

FIG. 9 is a flowchart illustrating an image display method according to some embodiments of the present

17

disclosure, this embodiment is a detailed description on the basis of the above embodiment. Optionally, the displaying the second blood vessel image and the first blood vessel image simultaneously includes: obtaining an updated first blood vessel image obtained by the angiography device based on a changed first horizon-of-view parameter when the first horizon-of-view parameter corresponding to the first blood vessel image changes ; determining an updated second horizon-of-view parameter based on the changed first horizon-of-view parameter and the parameter adjustment instruction, and simultaneously displaying the updated second blood vessel image determined based on the updated second horizon-of-view parameter and the updated first blood vessel image.

The specific implementation steps of this embodiment include:

S 910, obtaining a first blood vessel image of an imaging object obtained by the angiography device based on a first horizon-of-view parameter, and displaying the first blood vessel image.

In one embodiment, in combination with embodiment one, step S910 specifically includes that: the imaging object is photographed by using the angiographical device during a photography cycle, first fluoroscopic data of the imaging object irradiated by the angiographical device is obtained at a first energy based on the first horizon-of-view parameter, and during the photography cycle, and second fluoroscopic data of the imaging object irradiated by the radiation source at a second energy different from the first energy is obtained. Afterwards, the first blood vessel image of the imaging object is determined based on the first fluoroscopic data and the second fluoroscopic data, and the first blood vessel image is displayed.

In another embodiment, in combination with embodiment two, step S910 specifically includes that: a virtual human body model corresponding to the imaging object is obtained. The virtual human body model is displayed on an interactive interface, and first model information corresponding to a user operation instruction is displayed on the virtual human body model. Based on the first model information, an internal human body image corresponding to the first model information in the virtual human body model is determined, and the internal human body image is displayed. If the internal human body image corresponding to the first model information corresponds to a position of the first blood vessel of a target photography, the position of the first blood vessel corresponding to the medical imaging device is determined based on the first model information. If the internal human body image corresponding to the first model information does not correspond to the position of the first blood vessel of the target photography, the second model information different from the first model information is continued to be obtained, and the position of the first blood vessel corresponding to the medical imaging device is determined based on the internal human body image corresponding to the second model information. After determining the position of the first blood vessel, the first blood vessel image of the imaging object obtained by the angiography device based on the first horizon-of-view parameter is obtained, and the first blood vessel image is displayed.

S 920, determining a second horizon-of-view parameter based on a received parameter adjustment instruction and the first horizon-of-view parameter, and determining a second blood vessel image based on the second horizon-of-view parameter.

In one embodiment, in combination with embodiment one, step S920 is specifically: the second horizon-of-view parameter is determined based on the received parameter adjustment instruction and the first horizon-of-view parameter, and the imaging object is photographed by using the angiography device during a photography cycle, third fluoroscopic data of the angiography device irradiating the imaging object at the first energy is obtained based on the second horizon-of-view parameter, and during the photography process, and fourth fluoroscopic data of the radiation source irradiating the imaging object at the second energy different from the first energy is obtained. Afterwards, the second blood vessel image of the imaging object is determined based on the third fluoroscopic data and the fourth fluoroscopic data, and the second blood vessel image is displayed.

In another embodiment, in combination with embodiment two, step S920 specifically: the virtual human body model corresponding to the imaging object is obtained. The virtual human body model is displayed on the interactive interface, and the first model information corresponding to the user operation instruction is displayed on the virtual human body model. Based on the first model information, the internal human body image corresponding to the first model information in the virtual human body model is determined, and the internal human body image is displayed. If the internal human body image corresponding to the first model information corresponds to the position of the second blood vessel of the target photography, the position of the second blood vessel corresponding to the medical imaging device is determined based on the first model information. If the internal human body image corresponding to the first model information does not correspond to the position of the second blood vessel of the target photography, the second model information different from the first model information is continued to be obtained, and the position of the second blood vessel corresponding to the medical imaging device is determined based on the internal human body image corresponding to the second model information. After determining the position of the second blood vessel, the first blood vessel image of the imaging object obtained by the angiography device based on the first horizon-of-view parameter is obtained, and the first blood vessel image is displayed.

In one embodiment, optionally, the imaging object includes a blood vessel and a catheter inserted into the blood vessel, and the determining a second horizon-of-view parameter based on the received parameter adjustment instruction and the first horizon-of-view parameter further includes: obtaining a catheter tip image corresponding to the catheter in the first blood vessel image when the scaling adjustment ratio is greater than one, taking a position of a horizon-of-view center and a horizon-of-view range corresponding to the catheter tip image as a position of a second horizon-of-view center and a second horizon-of-view range, respectively, in the second horizon-of-view parameter.

The angiography technique is a technique in which a contrast agent is injected into a target blood vessel by means of a catheter intervention. Specifically, in the angiography technique, a catheter is inserted into a blood vessel in order to deliver the contrast agent through the catheter to the designated vessel position after the contrast agent is injected into the catheter. During the catheter intervention, a real-time imaging of the blood vessel is required in order to observe the position of the catheter in the blood vessel. In this embodiment, the first blood vessel image includes a catheter image. Specifically, the catheter image includes a catheter tip image, which is an image obtained after imaging the tip of the catheter.

The catheter tip image corresponding to the catheter in the first blood vessel image is obtained, specifically, the catheter tip image is obtained by dividing the first blood vessel image based on a preset division rule, exemplarily, the preset division rule includes a preset image size and a preset image shape, etc. By way of example, the preset image shape may be a square, a rectangle, a circle, or an irregular shape. The preset image size may be determined according to the preset image shape, exemplarily, when the preset image shape is a square, the preset image size may be 10mm*10mm, and when the preset image shape is a circle, the preset image size may be a radius of 5mm. Of course, the preset image size may also be an image area of the catheter tip image.

The advantage of this setup is that by automatically recognizing the catheter tip image in the first blood vessel image, the step of manually selecting a region of interest image by human is avoided. When the first blood vessel image is changed, a real-time tracking and recognition of the catheter tip image in the changed first blood vessel image can also be achieved, thus greatly improving the diagnostic efficiency of the subsequent angiography process of angiography.

On the basis of the above embodiment, optionally, the determining a second blood vessel image based on the second horizon-of-view parameter includes: obtaining a second blood vessel image obtained by the angiography device based on the second horizon-of-view parameter; or determining a reference blood vessel image in the first blood vessel image based on the position of the second horizon-of-view center and the second horizon-of-view range in the second horizon-of-view parameter when the scaling adjustment ratio is greater than one, and determining the second blood vessel image based on the second magnification in the second horizon-of-view parameter and the reference blood vessel image.

Whether the scaling adjustment ratio is greater than one or less than one, the angiography device may be controlled to obtain the second blood vessel image based on the second horizon-of-view parameter. In particular, when the scaling adjustment ratio is less than one, it means that a zooming out operation is performed on the first blood vessel image, at which time the second horizon of view is usually larger than the first horizon of view, i.e., the first blood vessel image does not contain all image information required for the second blood vessel image, and the second blood vessel image needs to be obtained by the angiography device based on the second horizon-of-view parameter again. In another embodiment, when the scaling adjustment ratio is greater than one, it means that a magnification operation is performed on the first blood vessel image, at which time the first blood vessel image usually contains all the image information required for the second blood vessel image. Of course, there may be cases where the first blood vessel image does not usually contain all the image information required for the second blood vessel image, such as when the position of the second horizon-of-view center is a boundary point of the first blood vessel image and the second horizon-of-view range is not 0. In this case, the method of controlling the second blood vessel image obtained by the angiographical device based on the second horizon-of-view parameter may also be used. In this embodiment, for example, the first blood vessel image contains all the image information required for the second blood vessel image, the reference blood vessel image contains all the image information required for the second blood vessel image, and a magnification corresponding to the reference blood vessel image is the first magnification, so it is also necessary to perform a magnification operation on the reference blood vessel image based on the second magnification in the second horizon-of-view parameter to obtain a second display image. Exemplarily, the algorithm during performing the magnification operation includes, but is not limited to, at least one of a nearest neighbor interpolation algorithm, a bilinear interpolation algorithm, and a higher-order interpolation algorithm.

The advantage of such a setup is that by intercepting the reference blood vessel image directly in the first blood vessel image and obtaining the second blood vessel image directly based on the reference blood vessel image and the second horizon-of-view parameter, aa number of repeated imaging and radiation dose are further reduced and the service life of the angiography device is improved.

S 930, displaying the second blood vessel image simultaneously with the first blood vessel image.

In one embodiment, optionally, the displaying the second blood vessel image simultaneously with the first blood vessel image includes: displaying the second blood vessel image simultaneously with the first blood vessel image. The advantage of such a setup is that the user is prevented from repeatedly switching between displaying the first blood vessel image and the second blood vessel image.

S 940, obtaining an updated first blood vessel image obtained by the angiography device based on a changed first horizon-of-view parameter when the first horizon-of-view parameter corresponding to the first blood vessel image changes.

S 950, determining an updated second horizon-of-view parameter based on the changed first horizon-of-view parameter and the parameter adjustment instruction.

In the case of the parameter adjustment instruction remains unchanged, when the first horizon-of-view parameter changes, the first blood vessel image also changes, while the second horizon-of-view parameter is changed following the first horizon-of-view parameter to obtain the updated second horizon-of-view parameter.

S 960, simultaneously displaying the updated second blood vessel image determined based on the updated second horizon-of-view parameter and the updated first blood vessel image.

Optionally, based on the above embodiment, the displaying the second blood vessel image simultaneously with the first blood vessel image includes: displaying the second blood vessel image in a display region different from the first blood vessel image on a display device corresponding to the first blood vessel image; or displaying the second vessel image on a display device different from the first vessel image. Specifically, in one embodiment, the display interface of the same display device includes at least two regions of display images, wherein one display region is for displaying a first display image and the other display region is for displaying a second display image. In another embodiment, the angiographical device includes at least two display devices, wherein one display device is used to display the first display image and the other display device is used to display the second display image.

Of course, when both the first horizon-of-view parameter and the second horizon-of-view parameter are changed, the updated first blood vessel image and the updated second blood vessel image obtained may also be displayed in different display regions of the display device, or, alternatively, may be displayed on different display devices.

The technical solution of this embodiment, by determining the updated second horizon-of-view parameter based on the changed first horizon-of-view parameter and the parameter adjustment instruction, and displaying the updated second-vessel image determined based on the updated second horizon-of-view parameter simultaneously with the updated first blood vessel image, solves the step of re-determining the second blood vessel image when the first blood vessel image is changed by the user, improves the diagnostic efficiency of angiography, and also avoids the positioning error caused by human factors in the process of repeated confirmation.

Embodiment four

Breast cancer is an important disease that poses a serious threat to women's health worldwide. The mammography is now recognized as a preferred screening modality for breast cancer. With the continuous updating of imaging devices, a digital breast tomosynthesis technique, also known as a digital breast tomosynthesis (DBT), emerged. The DBT is a three-dimensional imaging technique that allows projection data of the breast to be obtained at different angles during a short scan, and then reconstructs these separate images into a 3D tomosynthesis containing a series of high-resolution 3D tomosynthesis images of the breast. These tomosynthesis images are displayed individually or dynamically in a continuous playback format. Each tomosynthesis shows the structure of each section of the breast, and the entire breast 3D tomosynthesis represents the reconstructed breast.

In the prior art, some projection methods (e.g., a maximum density projection, an average projection, etc.) are usually used to obtain a 2D image similar to a 2D digital mammography image on a 3D tomographic image. However, after scanning by the DBT imaging system, due to a small scanning angle and poor Z-axis resolution, only a good image can be obtained in a direction parallel to the detector, but not in other directions, which is not conducive to the user to observe the overall distribution of lesions and lesion localization.

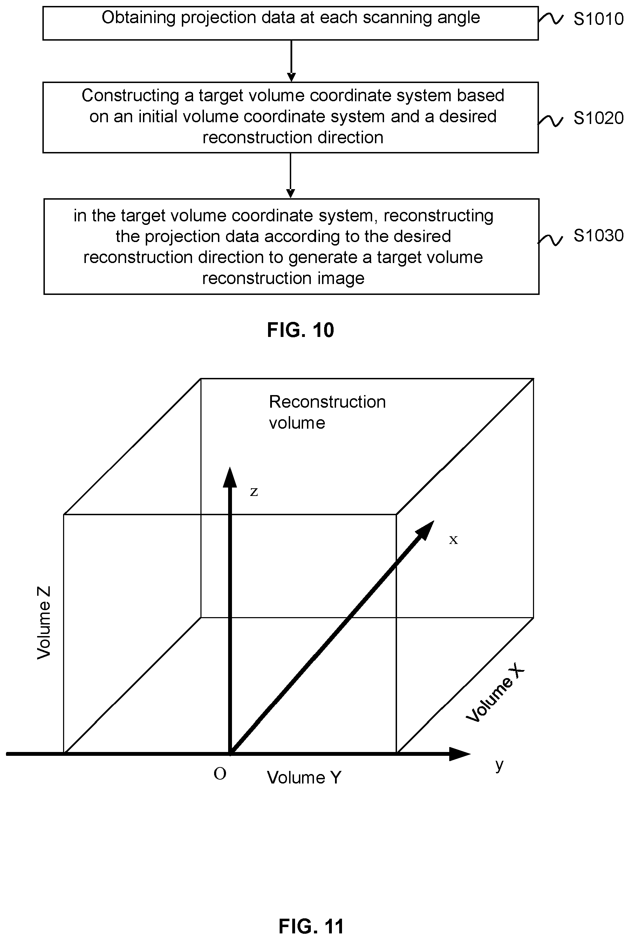

FIG. 10 is a schematic flowchart illustrating a method for generating a volume reconstruction image according to some embodiments of the present disclosure, this embodiment can be applied to the case of determining a target volume reconstruction image in a desired reconstruction direction, with the advantage that the target volume reconstruction image in the desired reconstruction direction and in a direction perpendicular to the desired reconstruction direction can be obtained, i.e., image information of the target volume reconstruction image is rich and comprehensive, which facilitates the user to quickly locate a region of interest on the target volume reconstruction image, and the method can be performed by a device for generating a volume reconstruction image, wherein the device may be implemented by software and/or hardware and is generally integrated in a terminal or control device. Referring specifically to FIG. 10, the method may include the following steps:

S 1010, obtaining projection data at each scanning angle.

Optionally, a tube may be controlled by the image acquisition device to emit X-rays at different angles to a scanned object, the different angles being the scanning angles. The projection data may be data received by the detector of the image acquisition device. Exemplarily, the image acquisition device in this embodiment may be a digital breast tomosynthesis (DBT) device, wherein a tube of the DBT device emits X-rays to the scanned object, receives the X-rays through the scanned object via a detector and converts the X-rays through the scanned object into digital projection data. The projection data at each scanning angle is sent to the control device. Optionally, the scanning angle of the tube of the digital mammography device is in a range of 15 degrees.

In one implementation, a first horizon-of-view parameter of each scanning angle is determined for each scanning angle, and a second horizon-of-view parameter of each scanning angle is determined based on a parameter adjustment instruction of each scanning angle and the first horizon-of-view parameter of each scanning angle. Afterwards, a first projection image of each scanning angle is obtained based on the first horizon-of-view parameter of each scanning angle and a second projection image is obtained based on the second horizon-of-view parameter of each scanning angle, and projection data at each scanning angle is determined based on the first projection image and the second projection image of each scanning angle.

Specifically, based on the first projection image and the second projection image of each scanning angle, the first projection image and the second projection image of a certain scanning angle may be weighted to determine projection data at that scanning angle.

S 1020, constructing a target volume coordinate system based on an initial volume coordinate system and a desired reconstruction direction.

The desired reconstruction direction may be a vector of any direction in the initial volume coordinate system, which may vary with a scanning angle. The embodiment uses a volume reconstruction technique to volumetrically reconstruct the projection data, constructs a three-dimensional structure model of the projection data, and in the constructed three-dimensional structure model, obtains a vector of any direction of an external input and uses the vector as the desired reconstruction direction to volumetrically reconstruct the projection data based on the desired reconstruction direction and the initial volume coordinate system, so as to facilitate to visually display projection information that is not visible to the naked eye in a form of a tomographic image.

The volume reconstruction technique can be understood as a body mapping technique by specifying a resistivity of each voxel in a 3D structural model and considering a transmission, a reflection and a reflection effect of each voxel on a light. The transmission of the light depends on an opacity of the voxel; the reflection of the light depends on a materiality of the voxel, the greater the materiality is, the stronger the reflected light is; the reflection of the light depends on an angle between a surface of the voxel and an incident light. In principle, the steps of the body mapping are divided into four steps: projection, defocusing, rendering and synthesis. Body mapping algorithm may be divided into a spatial domain method and a transform domain method according to different processing data domains; the spatial domain method is to process and display original spatial data directly, and the transform domain method is to transform body data to a transform domain and then process and display it.

The method of determining the initial volume coordinate system includes: performing a volume reconstruction of the projection data to obtain an initial volume reconstruction image, constructing the initial volume coordinate system based on the initial volume reconstruction image. Optionally, a first coordinate original point of the initial volume coordinate system is a point at any position on an edge length of a reconstructed volume at a first longitudinal axis corresponding to the initial volume reconstruction image; a first horizontal axis is in a plane belonging to the first longitudinal axis, starting at the first coordinate original point and perpendicular to the first longitudinal axis; and the first vertical axis starts at the first coordinate original point and is perpendicular to the plane where the first horizontal axis and the first longitudinal axis are located. Optionally, the first coordinate original point may be a vertex, a midpoint or a third of the edge length of the reconstructed volume at the first longitudinal axis, etc. This embodiment takes the midpoint of the edge length at the first longitudinal axis as the first coordinate original point.

The reconstructed volume (volume) in FIG. 11 shows a schematic diagram of the definition of the initial volume coordinate system, and the reconstructed volume (volume) in FIG. 11 can be the above-mentioned three-dimensional structure model, and after the control device obtains the projection data, it uses the volume reconstruction technique to reconstruct a volume of the projection data to obtain the initial volume reconstruction image and the reconstructed volume (volume), and constructs the initial volume coordinate system based on the initial volume reconstruction image. Specifically, the first coordinate original point O of the initial volume coordinate system in FIG. 11 is the midpoint of the edge length (VolumeY) to which the first longitudinal axis Y of the reconstructed volume (volume) belongs, the first horizontal axis X is on a bottom surface of the edge length to which the first longitudinal axis Y belongs, parallel to the edge length (VolumeY), and the initial volume coordinate system is constructed based on the determined first coordinate original point O, the first horizontal axis X, the first longitudinal axis Y and the first vertical axis Z.

Further, the initial volume coordinate system is constructed by the above steps and the target volume coordinate system is constructed in combination with the desired reconstruction direction. Optionally, the method of constructing a target volume coordinate system includes: determining a reconstructed volume to which the initial volume coordinate system belongs; determining, within the reconstructed volume, a first current plane based on the desired reconstruction direction, in a direction perpendicular to the desired reconstruction direction; constructing a second current plane based on the first longitudinal axis and the first vertical axis, taking a midpoint of an intersection of the second current plane and the first current plane as a second coordinate original point of the target volume coordinate system, and taking the desired reconstruction direction as a second vertical axis of the target volume coordinate system.

The first current plane is a plane in which any one of the tomographic images of the aforementioned three-dimensional structural model is located. Optionally, the method of determining the reconstructed volume to which the initial volume coordinate system belongs is to intercept $\dfrac{D}{2}$ in a positive coordinate axis and a negative coordinate axis of the first vertical axis Y respectively, intercept D in a positive coordinate axis of the first horizontal axis X and the first vertical axis Z respectively, construct a square according to the above intercepted distance, and use the constructed square as the reconstructed volume.

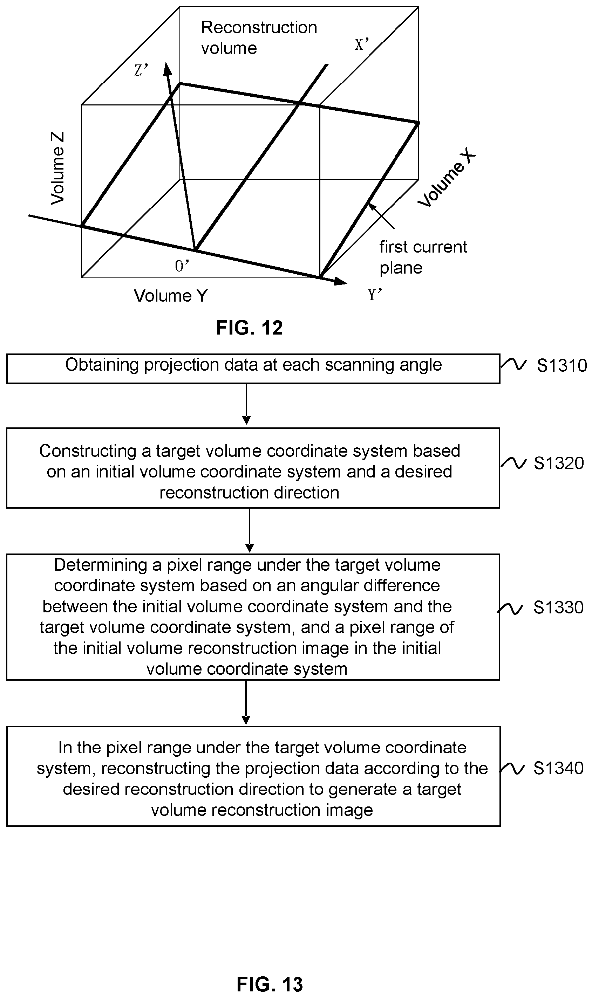

Exemplarily, the process of determining the target volume coordinate system is explained in connection with FIGs. 11 and 12. The desired reconstruction direction is obtained, the first current plane is determined perpendicular to the desired reconstruction direction, the second current plane is constructed based on the first longitudinal axis Y and the first vertical axis Z, the midpoint of the intersection line between the second current plane and the first current plane is taken as the second coordinate original point of the target volume coordinate, and the desired reconstruction direction is taken as the second vertical axis Z' in FIG. 12. Further, in the first current plane, the edge length belonging to the second coordinate original point is taken as the second longitudinal axis Y' of the target volume reconstruction system, and the vector of the plane starting at the second coordinate original point and perpendicular to the second vertical axis Y' and the second vertical axis Z' is taken as the second horizontal axis X'.

S 1030, in the target volume coordinate system, reconstructing the projection data according to the desired reconstruction direction to generate a target volume reconstruction image.

Optionally, the projection data may be reconstructed by using an iterative reconstruction algorithm, a filtered inverse projection reconstruction algorithm, an inverse projection filtered reconstruction algorithm, etc., to generate a target volume reconstruction image. The target volume reconstruction image may be perpendicular to the desired reconstruction direction, and may also be at other angles to the desired reconstruction direction.

It can be understood that after the control device obtains the projection data at different scanning angles, it can determine each desired reconstruction direction corresponding to each scanning angle, and reconstruct the projection data according to each desired reconstruction direction to obtain a target volume reconstruction image perpendicular to the desired reconstruction direction or at other angles to the desired reconstruction direction. Compared with the prior art in which the volume reconstruction image with better resolution can be obtained only in the direction parallel to the detector due to the constant vertical axis direction of the initial volume coordinate system and cannot obtain information in other directions, this embodiment can obtain volume reconstruction images with better resolution in multiple directions by changing the desired reconstruction direction and reconstructing according to different desired reconstruction directions, which is conducive to the user's analysis of the volume reconstruction images in multiple directions and target positioning.

This embodiment provides a technical solution to generate a target volume reconstruction image by obtaining the projection data at each scanning angle, constructing a target volume coordinate system based on the initial volume coordinate system and the desired reconstruction direction, and reconstructing the projection data according to the desired reconstruction direction under the target volume coordinate system. The problem that only a better image can be obtained in the direction parallel to the detector but not in other directions in the prior art is solved. By setting different desired reconstruction directions, the purpose of reconstructing in different desired reconstruction directions and obtaining a better resolution volume reconstruction image in each direction is achieved, which facilitates the user to effectively analyze the volume reconstruction images in multiple reconstruction directions and perform a target positioning.

FIG. 13 is a schematic flowchart illustrating a method for generating a volume reconstruction image according to some embodiments of the present disclosure. The technical solution of this embodiment is refined on the basis of the above embodiment. Optionally, the reconstructing the projection data according to the desired reconstruction direction under the target volume coordinate system to generate a target volume reconstruction image includes: determining a pixel range under the target volume coordinate system based on an angular difference between the initial volume coordinate system and the target volume coordinate system, and a pixel range of the initial volume reconstruction image in the initial volume coordinate system; reconstructing the projection data according to the desired reconstruction direction within the pixel range under the target volume coordinate system to generate the target volume reconstruction image. Please refer to the above embodiments for those portions of the embodiment of the method that are not described in detail. Referring specifically to FIG. 13, the method may include the following steps:

S 1310, obtaining projection data at each scanning angle.

S 1320, constructing a target volume coordinate system based on an initial volume coordinate system and a desired reconstruction direction.

S 1330, determining, based on an angular difference between the initial volume coordinate system and the target volume coordinate system, and a pixel range of the initial volume reconstruction image in the initial volume coordinate system, a pixel range under the target volume coordinate system.

The method as described in the preceding embodiment determines the initial volume coordinate system and the target volume coordinate system, and the control device determines the coordinate information of the first coordinate original point 0 and the second coordinate original point 0', the angular difference between the first horizontal axis *X* and the second horizontal axis *X',* the angular difference between the first longitudinal axis *Y* and the second longitudinal axis *Y',* and the angular difference between the first vertical axis Z and the second vertical axis Z', respectively, i.e., the angular difference between the initial volume coordinate system and the target volume coordinate system. In addition, the control device determines a pixel range of the initial volume reconstruction image in each direction of the initial volume coordinate system based on the pixel range of the initial volume reconstruction image, and further determines a pixel range of the initial volume reconstruction image in the determined target volume coordinate system by combining the angular difference between the initial volume coordinate system and the target volume coordinate system.

Explained specifically in relation to the schematic diagrams shown in FIGs. 11 and 12, the dimensions of each edge length of the reconstructed volume shown in FIGs. 11 and 12 are *VolumeX, VolumeY* and *VolumeZ,* respectively, and the first coordinate original point 0 of the initial volume coordinate system is a midpoint of the edge length of the reconstructed volume (volume) at the first longitudinal axis Y. A pixel range of the initial volume reconstruction image in the direction of the first horizontal axis *X* of the initial volume coordinate system is *PixelSizeX,* a pixel range of the initial volume reconstruction image in the direction of the first longitudinal axis Y of the initial volume coordinate system is *PixelSizeY,* and a pixel range of the initial volume reconstruction image in the direction of the first vertical axis Z of the initial volume coordinate system is *PixelSizeZ.* Based thereon, the first horizontal axis is defined as: *ReconCoordinateX, [0: VolumeX] * PixelSizeX,* the first longitudinal axis is defined as: *ReconCoordinateY,*

$$\left[-\frac{VolumeY}{2}:\frac{VolumeY}{2}\right] * PixelSizeY$$

, and the first vertical axis is defined as. *ReconCoordinateZ, [0: VolumeZ] * PixelSizeZ;* wherein the *ReconCoordinateX* denotes the X-axis of the initial volume coordinate system, the *ReconCoordinateY* denotes the *Y*-axis of the initial volume reconstruction coordinate system, and the *ReconCoordinateZ* denotes the Z-axis of the initial volume reconstruction coordinate system.

Accordingly, the second coordinate original point 0' is a midpoint of the intersection line between the second current plane and the first current plane, the second horizontal axis *X'* is defined as: *ReconCoordinateXNew,* [0: *VolumeX*/cosβ] * *PixelSizeX,* the second longitudinal axis *Y'* is defined as: *ReconCoordinateYNew,* [0 -

$$\frac{VolumeY}{2}:VolumeY/\cos\alpha - \frac{VolumeY}{2}] * PixelSizeY$$

, and the desired reconstruction direction Z' (i.e., the second vertical axis) is defined as: *ReconCoordinateZNew(z) = ReconCoordinateYNew(y) * sinα + ReconCoordinateYNew(x) * sinβ+ ReconCoordinateZ(z)/cosθ.*

α denotes an angle between the second longitudinal axis *Y'* and the first longitudinal axis *Y,* β denotes an angle between the second horizontal axis *X'* and the first horizontal axis X, and θ denotes an angle between the second vertical axis Z' and the first vertical axis Z; the *ReconCoordinateXNew* denotes the X-axis of the target volume coordinate system (i.e., the second horizontal axis X'), the *ReconCoordinateYNew* denotes the *Y*-axis of the target volume coordinate system (i.e. the second longitudinal axis *Y'),* the *ReconCoordinateZNew(z)* denotes the Z-axis of the target volume coordinate system (i.e. the second vertical axis Z'); [0: VolumeX/ cosβ] * *PixelSizeX* denotes the *X* -axis of the target volume coordinate system (i.e., the second horizontal axis *X')* of the target volume coordinate system, [0 -

$$\frac{VolumeY}{2}:VolumeY/\cos\alpha - \frac{VolumeY}{2}] * PixelSizeY$$

denotes a pixel range on the *Y*-axis (i.e., the second longitudinal axis Y') of the target volume coordinate system, and a pixel range on the Z-axis (i.e., the second vertical axis Z') of the target volume coordinate system is 0.

S 1340, in the pixel range under the target volume coordinate system, reconstructing the projection data according to the desired reconstruction direction to generate a target volume reconstruction image.

The pixel range included in the target volume coordinate system is determined by the preceding steps, and the target volume reconstruction image may be generated by reconstructing the projection data at the pixel range and using an iterative reconstruction algorithm, a filtered inverse projection reconstruction algorithm, an inverse projection filtered reconstruction algorithm, or the like. The target volume reconstruction image may be perpendicular to the desired reconstruction direction, and may also be at other angles to the desired reconstruction direction.

Optionally, before the reconstructing the projection data further includes: pre-processing the projection data. The pre-processing includes at least one of an image segmentation, a gray value transformation, and a window width and window position transformation. The image segmentation may employ a threshold-based image segmentation, region growing, etc., for filtering the projection data to reduce a calculation volume of the volume reconstruction; the gray value transformation may employ an image inversion, a logarithmic transformation, and a gamma transformation, etc., for improving a contrast of the projection data, which is conducive to improving a contrast of the volume reconstruction image and facilitating a user to analyze the reconstruction image; the window width and window position transformation may be used to increase a window width, decrease a window width or transform a window center point, which can remove noisy data from the projection data and help to improve the reconstruction efficiency of the projection data.

Optionally, after determining the target volume reconstruction image, the control device may further obtain an image rendering instruction of the target volume reconstruction image; render pixel points of the target volume reconstruction image based on the image rendering instruction, and display the target volume reconstruction image after rendering. For example, pixel values of pixel points within a region of interest determined by the user are increased to highlight the image of interest and facilitate analysis of the image by the user.

This embodiment provides a technical solution for generating a target volume coordinate system by determining a pixel range under the target volume coordinate system based on an angular difference between the initial volume coordinate system and the target volume coordinate system, and a pixel range of the initial volume reconstructed image under the initial volume coordinate system, and reconstructing the projection data according to the desired reconstruction direction within the pixel range under the target volume coordinate system to generate the target volume reconstruction image. The pixel range under the target volume coordinate system can be accurately determined, which further facilitates the generation of a volume reconstruction image with good resolution in each direction.

Embodiment five

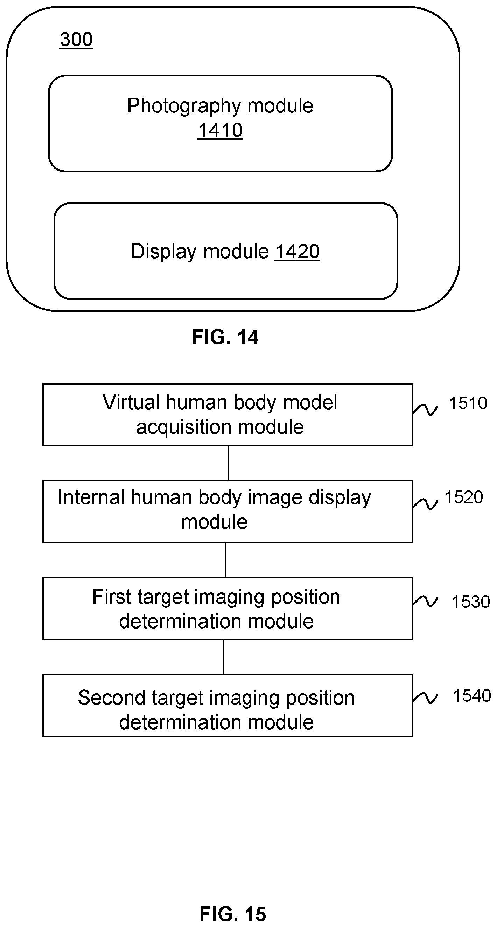

FIG. 14 is a diagram illustrating an exemplary module of a system for a dynamic fluoroscopy of a C-shaped arm device according to some embodiments of the present disclosure. As shown in FIG. 14, the system 1400 for a dynamic fluoroscopy may include a photography module 1410 and a display module 1420. In some embodiments, the system 1400 for a dynamic fluoroscopy may be implemented by the system 100 for a dynamic fluoroscopy shown in FIG. 1 (e.g., the processing device 140).

The photography module 1410 may be used to photograph a subject during a photography cycle, obtain, during the photography cycle, first fluoroscopic data of a radiation source irradiating the subject at a first energy and second fluoroscopic data of the radiation source irradiating the subject at a second energy different from the first energy. In some embodiments, the photography module 1410 may also be used to perform the photography of the subject during multiple successive photography cycles.

The display module 1420 may be used to display a dynamic image of the subject based on the first fluoroscopic data and the second fluoroscopic data obtained in each of the multiple successive photography cycles.

In other embodiments of the present disclosure, there is provided a device for a dynamic fluoroscopy, comprising at least one processor 140 and at least one memory device 150, the memory device 150 being used to store instructions that, when the at least one processor 140 executes at least some of the computer instructions to implement the method for a dynamic fluoroscopy.

In some further embodiments of the present disclosure, there is provided a computer-readable memory medium, the computer-readable memory medium stores computer instructions, and when the computer instructions are read by a computer (e.g., processing device 140), the processing device 140 executes the method 200 for a dynamic fluoroscopy.

It should be noted that the above description of the system for a dynamic fluoroscopy and its devices/modules is for descriptive convenience only and does not limit the present disclosure to the scope of the cited embodiments. It can be understood that for those skilled in the art, after understanding the principle of the system, it may be possible to make any combination of the individual devices/modules or form a subsystem to connect with other devices/modules without departing from this principle. For example, the photography module 1410 and the display module 1420 disclosed in FIG. 14 may be different modules in a single device (e.g., the processing device 140), or one module may implement the functions of two or more of the above-mentioned modules. For example, the photography module 1410 and the display module 1420 may be two modules, or one module may have the function of both photographing and displaying moving images. As another example, each module may have its own memory module. Further, for example, the individual modules may share a common memory module. Furthermore, for example, the photography module 1410 may include a first photography sub-module and a second photography sub-module, wherein the first photography sub-module may be used to obtain first perspective data of the subject irradiated by the radiation source at a first energy during the photography, and the second photography sub-module may be used to obtain second perspective data of the subject irradiated by the radiation source at a second energy. All variations such as these are within the scope of protection of the present disclosure.

Possible beneficial effects of embodiments of the present disclosure include, but are not limited to, (1) helping medical personnel to quickly understand the dynamic changes occurring in the subject's lesions and/or various tissues and organs over multiple photography cycles; and (2) being able to obtain the desired category of images using dual-energy subtraction techniques. It should be noted that different embodiments may produce different beneficial effects, and in different embodiments, the possible beneficial effects may be any one or a combination of the above, or any other beneficial effect that may be obtained.

Embodiment six

FIG. 15 is a schematic diagram illustrating an imaging positioning device for a medical imaging device according to some embodiments of the present disclosure. This embodiment may be applicable to the positioning of a target site, which may be implemented in software and/or hardware, and the imaging and positioning device includes: a virtual human body model acquisition module 1510, an internal human body image display module 1520, a first target imaging position determination module 1530, and a second target imaging position determination module 1540.

The virtual human body model acquisition module is used to obtain a virtual human body model corresponding to an imaging object and obtain first position information corresponding to a user operation instruction.

The internal human body image display module 1520 may be used to determine, based on the first position information, an internal human body image corresponding to the first position information in the virtual human body model, and display the internal human body image.

The first target imaging position determination module 1530 may be used to determine, based on the first position information, a target imaging position corresponding to the medical imaging device, if the internal human body image corresponding to the first position information corresponds to a target photography position.

The second target imaging position determination module 1540 may be used to proceed to obtain a second position information different from the first position information and determine the target imaging position corresponding to the medical imaging device based on the internal human body image corresponding to the second position information, if the internal human body image corresponding to the first position information does not correspond to the target photography position.

The technical solution of this embodiment, by determining the internal human body image corresponding to the first position information input by the user based on the virtual human body model, and obtaining the target information through the internal human body image corresponding to the positioning instruction, solves the problem of radiation damage to the human body during the imaging operation, allowing the user to set any parameters in the process of imaging and positioning without causing any damage to the human body, which in turn also ensures the positioning accuracy.

On the basis of the above technical solutions, optionally, the first position information includes first model information corresponding to the virtual human body model or first device information corresponding to the medical imaging device, wherein there is an association relationship between the first model information and the first device information.

On the basis of the above technical solutions, optionally, the association relationship includes a position association relationship, and the device further includes:

A position association relationship determination module used to convert a relative position relationship of the medical imaging device and the imaging object into a position association relationship of the medical imaging device and the virtual human body model; wherein the position association relationship is used to characterize a relationship of position parameters between the first model information and the first device information.

On the basis of the above technical solutions, optionally, the association relationship further includes a horizon-of-field association relationship, and the device further includes:

A horizon-of-field association acquisition module used to obtain a horizon-of-view association relationship between the first model information and the first device information; wherein the horizon-of-view association relationship is used to characterize a relationship of horizon-of-view parameters between the first model information and the first device information.

On the basis of the above technical solution, optionally, the virtual human body model acquisition module 1510 is used to:

display the virtual human body model on an interactive interface when the first position information is first model information, and display the first model information corresponding to the user operation instruction on the virtual human body model.

On the basis of the above technical solution, optionally, the first model information includes a graphical marker.

On the basis of the above technical solution, optionally, the graphical marker performs at least one operation of selecting, moving, zooming in and zooming out based on the user operation instruction.

On the basis of the above technical solution, optionally, the device further includes:

when the first position information is the first model information, determining a first imaging position corresponding to the medical imaging device based on the first model information and the association relationship, and controlling the medical imaging device to move to the first imaging position.

On the basis of the above technical solution, optionally, the first target imaging position determination module 1530 includes:

a first target device information determination unit used to determine a target imaging position corresponding to the medical imaging device based on the first model information and the association relationship, and control the medical imaging device to move to the target imaging position, when the first position information is the first model information.

On the basis of the above technical solution, optionally, the device further includes:

an imaging operation execution module used to control an imaging component in the medical imaging device to perform an imaging operation based on the horizon-of-field parameters; wherein the horizon-of-field parameters include at least one of a source image distance, a source object distance, and a magnification.

On the basis of the above technical solutions, optionally, the internal human body image display module 1520 specifically used to

determine, when the first position information is first device information, first model information corresponding to the virtual human body model based on the first device information and the association relationship, and determine an internal human body image based on the first model information.

On the basis of the above technical solution, optionally, the first target imaging position determination module 1530 includes:

a second target device information determination unit used to take an imaging position in the first device information as the target imaging position corresponding to the medical imaging device, when the first position information is the first device information.

On the basis of the above technical solution, optionally, the virtual human body model acquisition module 1510 specifically used to

according to obtained height data corresponding to the imaging object, select a virtual human body model corresponding to the height data; wherein the virtual human body model includes a human body shape model and an internal human body model.

On the basis of the above technical solution, optionally, the internal human body model includes at least one of a blood vessel model, an organ model, a skeletal model and a muscle model.

On the basis of the above technical solution, optionally, the medical imaging device includes a digital X-ray photography device, a C-shaped arm X-ray device, a mammography machine, a computed tomography radiography device, a magnetic resonance device, a positron emission tomography PET device, a positron emission tomography and a computed tomography PET-CT device, a positron emission tomography and a magnetic resonance imaging PET-MR device, or a radiotherapy imaging RT device.

The imaging positioning device provided in the embodiment of the present disclosure may be used to perform the imaging positioning method provided in the embodiment of the present disclosure, with the corresponding functions and beneficial effects of the execution method.

It should be noted that the units and modules included in the above embodiment of the imaging positioning device are only divided according to the functional logic, but are not limited to the above division, as long as they can achieve the corresponding functions; in addition, the specific names of the functional units are only for mutual distinction, and are not used to limit the scope of protection of the present disclosure.

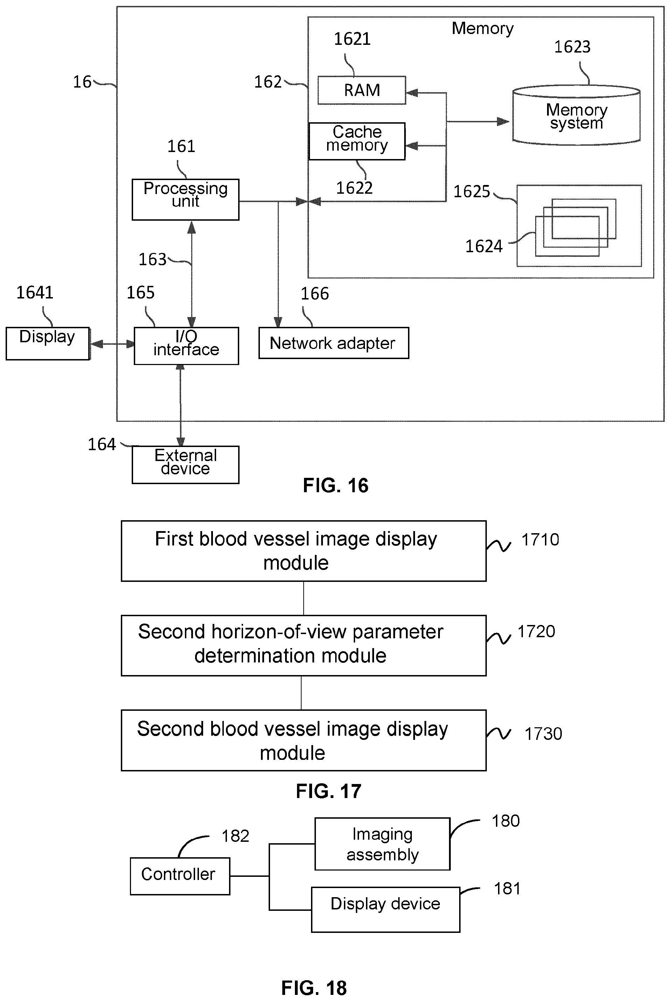

FIG. 16 is a schematic diagram illustrating a structure of a medical imaging device according to some embodiments of the present disclosure, embodiments of the present disclosure provide for the implementation of the imaging positioning method of the above embodiments of the present disclosure, and the imaging positioning device of the above embodiments may be configured. FIG. 16 illustrates a block diagram of an exemplary electronic device 16 suitable for use in implementing an embodiment of the present disclosure. The electronic device 16 shown in FIG. 16 is only an example and should not impose any limitations on the functionality and scope of use of the embodiments of the present disclosure.

As shown in FIG. 16, the electronic device 16 is represented as a general purpose computing device. Components of the electronic device 16 may include, but are not limited to: one or more processors or processing units 161, a system memory 162, a bus 163 connecting different system components (including a system memory 162 and a processing unit 161).

The bus 163 represents one or more of several types of bus architectures, including a memory bus or a memory controller, a peripheral bus, a graphic acceleration port, a processor, or a local area bus that uses any of the multiple bus architectures. Examples of these architectures include, but are not limited to, an industry standard architecture (ISA) bus, a microchannel architecture (MAC) bus, an enhanced ISA bus, a video electronics standards association (VESA) local area bus, and a peripheral component interconnect (PCI) bus.

The electronic device 16 typically includes multiple computer system readable media. These media may be any available media that may be accessible by the electronic device 16, including volatile and non-volatile media, removable and non-removable media.

The system memory 162 may include computer system readable media in the form of a volatile memory, such as a random access memory (RAM) 1621 and/or a cache memory 1622. The electronic device 16 may further include other removable/non-removable, volatile/non-volatile computer system memory media. By way of example only, the memory system 1623 may be used to read and write non-removable, non-volatile magnetic media (not shown in FIG. 16, commonly referred to as "hard disk drives"). Although not shown in FIG. 16, disk drives may be provided for reading and writing to removable non-volatile disks (e.g., "floppy disks"), and optical disk drives for reading and writing to removable non-volatile optical disks (e.g., CD-ROMs, DVD-ROMs, or other optical media). In these cases, each drive may be connected to the bus 163 through one or more data media interfaces. The system memory 162 may include at least one program product having a set (e.g., at least one) of program modules that are configured to perform the functions of each embodiment of the present disclosure.

A program/utility 1625 having a set of (at least one) program modules 1624 may be stored in, for example, the memory 162, such program modules 1624 including, but not limited to, an operating system, one or more applications, other program modules, and program data, each of these examples, or some combination thereof, may include an implementation of a network environment. The program modules 1624 typically perform the functions and/or methods in the embodiments described in the present disclosure.

The electronic device 16 may also communicate with one or more external devices 164 (e.g., a keyboard, a pointing device, a display 1641, etc.), and may also communicate with one or more devices that enable the user to interact with the electronic device 16, and/or with any device that enables the electronic device 16 to communicate with one or more other computing devices (e.g., a network card, a modem, etc.). Such communication may be via input/output (I/O) interface 165. And, the electronic device 16 may also communicate with one or more networks (e.g., a local area network (LAN), a wide area network (WAN), and/or a public network, such as the Internet) via a network adapter 166. As shown in FIG. 16, the network adapter 166 communicates with other modules of the electronic device 16 via the bus 163. It should be understood that, although not shown in the figures, other hardware and/or software modules may be used in conjunction with the electronic device 16, including but not limited to: a microcode, a device drive, a redundant processing unit, an external disk drive array, a RAID system, a tape drive, and a data backup memory system, etc.

The processing unit 161 performs various functional applications and data processing, such as implementing the imaging positioning method provided by the embodiment of the present disclosure, by running a program stored in the system memory 162.

In one embodiment, the electronic device 16 may be a terminal device, configured with an imaging positioning device, which is communicatively connected to an irradiation system. In another embodiment, the electronic device 16 may also be an irradiation system configured with an imaging positioning device.

Through the above equipment, the problem of radiation damage to the human body is solved, while ensuring the realization of imaging and positioning, so that the user can set any parameters in the process of imaging and positioning, and does not cause any damage to the human body, which in turn can also improve the positioning accuracy.

Embodiment five of the present disclosure also provides a memory medium comprising computer-executable instructions, wherein the computer-executable instructions, when executed by a computer processor, are used to perform an imaging positioning method, the method includes:

obtaining a virtual human body model corresponding to an imaging object and obtaining first position information corresponding to a user operation instruction;

determining, based on the first position information, an internal human body image corresponding to the first position information in the virtual human body model, and displaying the internal human body image;

determining, based on the first position information, a target imaging position corresponding to the medical imaging device, if the internal human body image corresponding to the first position information corresponds to a target photography position;

if the internal human body image corresponding to the first position information does not correspond to the target photography position, proceeding to obtain a second position information different from the first position information and determining the target imaging position corresponding to the medical imaging device based on the internal human body image corresponding to the second position information.

The computer memory medium of the embodiment of the present disclosure may employ any combination of one or more computer-readable media. The computer-readable medium may be a computer-readable signal medium or a computer-readable memory medium. The computer-readable memory medium may be, for example, but not limited to, an electrical, a magnetic, an optical, an electromagnetic, an infrared, or a semiconductor system, device, or instrument, or any combination of the above. More specific examples of computer-readable memory media (a non-exhaustive list) include: electrically connected with one or more wires, portable computer disks, hard disks, random access memory (RAM), read-only memory (ROM), erasable programmable read-only memory (EPROM or flash memory), optical fiber, portable compact disk read-only memory (CD-ROM), optical memory devices, magnetic memory devices, or any suitable combination of the above. For the purposes of this document, the computer-readable memory medium may be any tangible medium that contains or stores a program that may be used by or in combination with an instruction execution system, device, or instrument.

The computer-readable signal medium may include data signals propagated in a baseband or as part of a carrier wave that carries computer-readable program codes. Such propagated data signals may take multiple forms, including but not limited to an electromagnetic signal, an optical signal, or any suitable combination of the above. The computer-readable signal medium may also be any computer-readable medium other than a computer-readable memory medium that sends, propagates, or transmits a program for use by or in conjunction with an instruction execution system, device, or instrument.

The program codes contained on the computer-readable medium may be transmitted using any suitable medium, including but not limited to a wireless, a wire, a fiber optic cable, RF, etc., or any suitable combination of the above.

The computer program codes for performing the operations of the present disclosure may be written in one or more programming languages or combinations thereof, including object-oriented programming languages such as Java, Smalltalk, C++, and conventional procedural programming languages such as "C" or a similar programming languages. The program codes may be executed entirely on the user's computer, partially on the user's computer, as a stand-alone package, partially on the user's computer and partially on a remote computer, or entirely on a remote computer or server. In the case involving a remote computer, the remote computer may be connected to the user computer via any kind of network including a local area network (LAN) or a wide area network (WAN), or, alternatively, may be connected to an external computer (e.g., using an Internet service provider to connect via the Internet).

Of course, the memory medium comprising computer-executable instructions provided by the embodiment of the present disclosure, the computer-executable instructions are not limited to the method operations as described above, but can also perform the relevant operations in the imaging positioning method provided by any embodiment of the present disclosure.

Embodiment seven

FIG. 17 is a schematic diagram illustrating an image display device according to some embodiments of the present disclosure. This embodiment may be applicable to the use of an angiography device for imaging and displaying the imaging results, which may be implemented in software and/or hardware configured in the angiography device. The image display device includes: a first blood vessel image display module 1710, a second horizon-of-view parameter determination module 1720, and a second blood vessel image display module 1730.

The first blood vessel image display module 1710 is used to obtain a first blood vessel image of an imaging object obtained by the angiography device based on a first horizon-of-view parameter, and display the first blood vessel image.

The second horizon-of-view parameter determination module 1720 is used to determine a second horizon-of-view parameter based on a received parameter adjustment instruction and the first horizon-of-view parameter; wherein the parameter adjustment instruction includes a scaling adjustment ratio.

The second blood vessel image display module 1730 is used to determine a second blood vessel image based on the second horizon-of-view parameter and simultaneously display the second blood vessel image and the first blood vessel image.

The technical solution of this embodiment, by determining a second blood vessel image based on the received parameter adjustment instruction while displaying the first blood vessel image and displaying the second blood vessel image simultaneously with the first blood vessel image, solves the problem of repeatedly switching the display of scanned images of different horizons of view, reduces the count of repeated imaging and the amount of radiation, improves the service life of the angiography device, which in turn also improves the diagnostic efficiency of angiography and reduces the error of the diagnostic results of angiography caused by repeated switching.

On the basis of the above technical solution, optionally, the second horizon-of-view parameter determination module 1720 includes:

a second magnification determination unit used to determine a second magnification in the second horizon-of-view parameter based on the scaling adjustment ratio and the first magnification in the first horizon-of-view parameter.

On the basis of the above technical solution, optionally, the second horizon-of-view parameter determination module 1720 includes:

a second horizon-of-view range determination unit used to determine a second horizon-of-view range in the second horizon-of-view parameter based on the scaling adjustment ratio and a first horizon-of-view range in the first horizon-of-view parameter.

On the basis of the above technical solution, optionally, the imaging object includes a blood vessel and a catheter inserted into the blood vessel, the second horizon-of-view parameter determination module 1720 includes:

a second horizon-of-view center position determination unit used to obtain a catheter tip image corresponding to the catheter in the first blood vessel image when the scaling adjustment ratio is greater than one, take a position of a horizon-of-view center and a horizon-of-view range corresponding to the catheter tip image as a position of a second horizon-of-view center and a second horizon-of-view range, respectively, in the second horizon-of-view parameter.

On the basis of the above technical solution, optionally, the second blood vessel image display module 1730 includes:

a second blood vessel image determination unit used to obtain a second blood vessel image obtained by the angiography device based on the second horizon-of-view parameter; or determine a reference blood vessel image in the first blood vessel image based on the position of the second horizon-of-view center and the second horizon-of-view range in the second horizon-of-view parameter when the scaling adjustment ratio is greater than one, and determine the second blood vessel image based on the second magnification in the second horizon-of-view parameter and the reference blood vessel image.

On the basis of the above technical solution, optionally, the second blood vessel image display module 1730 includes:

a first display unit of the second blood vessel image used to display the second blood vessel image in a display region different from the first blood vessel image on a display device corresponding to the first blood vessel image; or display the second vessel image on a display device different from the first vessel image.

On the basis of the above technical solution, optionally, the second blood vessel image display module 1730 includes:

a display unit for updating the second blood vessel image used to obtain an updated first blood vessel image obtained by the angiography device based on a changed first horizon-of-view parameter when the first horizon-of-view parameter corresponding to the first blood vessel image changes; determine an updated second horizon-of-view parameter based on the changed first horizon-of-view parameter and the parameter adjustment instruction, and simultaneously display the updated second blood vessel image determined based on the updated second horizon-of-view parameter and the updated first blood vessel image.

The image display device provided in this embodiment of the present disclosure may be used to execute the image display method provided in this embodiment of the present disclosure, having the functions and beneficial effects corresponding to the execution method.

It should be noted that the units and modules included in the above embodiment of the image display device are only divided according to the functional logic, but are not limited to the above division, as long as they can achieve the corresponding functions; in addition, the specific names of the functional units are only for the convenience of mutual distinction, and are not used to limit the scope of protection of the present disclosure.

Embodiment eight

FIG. 18 is a schematic diagram illustrating a structure of an angiography device according to some embodiments of the present disclosure, the embodiment of the present disclosure provides for the implementation of the image display method described in any one of the above embodiments of the present disclosure, and the image display device in the embodiment of the present disclosure may be configured.

The angiography device comprises an imaging assembly 180, at least one display device 181 and a controller 182; wherein the imaging assembly 180 is configured to obtain a first blood vessel image based on a first horizon-of-view parameter; the display device 181 is configured to display the first blood vessel image and a second blood vessel image; the controller 182 includes one or more processors and a memory having one or more programs, which, when the one or more programs are executed by the one or more processors, causes the one or more processors to implement the image display method as described in any one of the above embodiments.

In one embodiment, optionally, the imaging assembly includes an X-ray source and a detector. The X-ray source is used to emit X-rays; and the detector, is used to generate a first blood vessel image based on the received X-rays.

In one embodiment, optionally, the X-ray source includes an X-ray high voltage generator and an X-ray tube. There are types of X-ray high voltage generators, such as a high frequency high voltage generator and a high frequency inverter high voltage generator, and the high frequency inverter high voltage generator is more commonly used. The high frequency inverter high voltage generator is divided into a continuous high frequency inverter high voltage generator and a computer-controlled pulsed high frequency inverter high voltage generator, in which the continuous high frequency inverter high voltage generator relies on a gate switch in the X-ray tube to generate a pulse wave during pulse acquisition. The X-ray tube may receive a high frequency high voltage output to generate X-rays, and specifications of the X-ray tube include both structural parameters and electrical parameters. The former refers to various parameters determined by the structure of the X-ray tube, such as a tilt angle of a target surface, an effective focal point, an external dimension, a weight, a filtering equivalent of the tube wall, an anode speed, an operating temperature and a form of cooling, etc. The electrical parameters are the specification data of the electrical performance of the X-ray tube, such as a filament heating voltage and current, a maximum tube voltage, a tube current, a maximum exposure time, a maximum allowable power and anode heat capacity, etc. The X-ray tube is characterized by a small focal point, a high heat capacity, a high load, a high rotational speed and a high heat dissipation rate. Exemplarily, a liquid metal bearing technology may be used to make the X-ray tube, which can avoid the wear and tear of bearings in the case of ordinary tubes at high rotational speed, not only increase the heat dissipation efficiency, but also improve the ability of the X-ray tube to withstand continuous load and improve the life of the tube, while reducing the intrinsic noise of the device and improving the signal-to-noise ratio of the image.

In one embodiment, optionally, the detector includes a panel detector.

Specifically, when the angiography device includes at least two display devices 181, a placement relationship between each display device and a placement position of each display device may be selected according to the user's usage habits. When displaying blood vessel images with different magnifications on the at least two display devices 181, magnification levels of the blood vessel images corresponding to the different display devices 181 may be preset, exemplarily, the magnification levels corresponding to display device A, display device B and display device C are reduced in order, i.e., the blood vessel image with the largest magnification is displayed on display device A, and the blood vessel image with the smallest magnification is displayed on display device C. The advantage of this setting is that if the correspondence between the blood vessel images and the display devices is not set, when there are many display devices, the user needs to check the blood vessel images on each display device one by one to find the desired target blood vessel image, thus increasing the burden of the user in finding the target blood vessel image. By setting the correspondence between the blood vessel images and the display devices, the user can follow the correspondence to find the target blood vessel images, thus greatly reducing the user's burden of finding the target blood vessel images and thus improving the diagnostic efficiency of angiography.

In one embodiment, optionally, the angiography device further includes a catheter for injecting a contrast agent into a target blood vessel.

The memory in the controller, as a computer-readable memory medium, may be used to store software programs, computer-executable programs, and modules, such as program instructions/modules corresponding to the image display method in this embodiment of the present disclosure (e.g., a first blood vessel image display module 1710, a second horizon-Of-view parameter determination module 1720, and a second blood vessel image display module 1730). The processor executes various functional applications of the angiographical device and data processing by running the software programs, instructions, and modules stored in the memory, i.e., to implement the image display method described above.

The memory may primarily include a stored program region and a stored data region, wherein the stored program region may store operating systems, applications required for at least one function, and the stored data region may store data created based on the use of the terminal, etc. In addition, the memory may include a highspeed random access memory, and may also include a non-volatile memory, such as at least one disk memory device, a flash memory device, or other non-volatile solid state memory device. In some embodiments, the memory may further include memories that are remotely located relative to the processor, and these remote memories may be connected to the angiographical device via networks. Examples of the networks include, but are not limited to, an Internet, an enterprise intranet, a local area network, a mobile communication network, and combinations thereof.

In one embodiment, optionally, the angiography device further includes an input device for receiving input numeric or character information and for generating a key signal input related to user settings of the angiography device and control of functions.

The above angiography device solves the problem of repeatedly switching the display of scanned images of different horizons, reduces the number of repeated imaging and the amount of radiation, improves the service life of the angiography device, and thus also improves the diagnostic efficiency of angiography and reduces the errors in the diagnostic results of angiography caused by repeated switching.

Embodiments of the present disclosure also provide a storage medium comprising computer-executable instructions, wherein the computer-executable instructions, when executed by a computer processor, are used to perform an image display method, the method includes:

obtaining a first blood vessel image of an imaging object obtained by the angiography device based on a first horizon-of-view parameter, and displaying the first blood vessel image;

determining a second horizon-of-view parameter based on a received parameter adjustment instruction and the first horizon-of-view parameter; wherein the parameter adjustment instruction includes a scaling adjustment ratio;

determining a second blood vessel image based on the second horizon-of-view parameter and simultaneously displaying the second blood vessel image and the first blood vessel image.

The non-transitory computer memory medium of the embodiment of the present disclosure may employ any combination of one or more computer-readable media. The computer-readable medium may be a computer-readable signal medium or a computer-readable memory medium. The computer-readable memory medium may be, for example, but not limited to, an electrical, a magnetic, an optical, an electromagnetic, an infrared, or a semiconductor system, device, or instrument, or any combination of the above. More specific examples of computer-readable memory media (a non-exhaustive list) include: electrically connected with one or more wires, portable computer disks, hard disks, random access memory (RAM), read-only memory (ROM), erasable programmable read-only memory (EPROM or flash memory), optical fiber, portable compact disk read-only memory (CD-ROM), optical memory devices, magnetic memory devices, or any suitable combination of the above. For the purposes of this document, the computer-readable memory medium may be any tangible medium that contains or stores a program that may be used by or in combination with an instruction execution system, device, or instrument.

The computer-readable signal medium may include data signals propagated in a baseband or as part of a carrier wave that carries computer-readable program codes. Such propagated data signals may take multiple forms, including but not limited to an electromagnetic signal, an optical signal, or any suitable combination of the above. The computer-readable signal medium may also be any computer-readable medium other than a computer-readable memory medium that sends, propagates, or transmits a program for use by or in conjunction with an instruction execution system, device, or instrument.

The program codes contained on the computer-readable medium may be transmitted using any suitable medium, including but not limited to a wireless, a wire, a fiber optic cable, RF, etc., or any suitable combination of the above.

The computer program codes for performing the operations of the present disclosure may be written in one or more programming languages or combinations thereof, including object-oriented programming languages such as Java, Smalltalk, C++, and conventional procedural programming languages such as "C" or a similar programming languages. The program codes may be executed entirely on the user's computer, partially on the user's computer, as a stand-alone package, partially on the user's computer and partially on a remote computer, or entirely on a remote computer or server. In the case involving a remote computer, the remote computer may be connected to the user computer via any kind of network including a local area network (LAN) or a wide area network (WAN), or, alternatively, may be connected to an external computer (e.g., using an Internet service provider to connect via the Internet).

Of course, the memory medium comprising computer-executable instructions provided by the embodiment of the present disclosure, the computer-executable instructions are not limited to the method operations as described above, but can also perform the relevant operations in the image display method provided by any embodiment of the present disclosure.

Embodiment nine

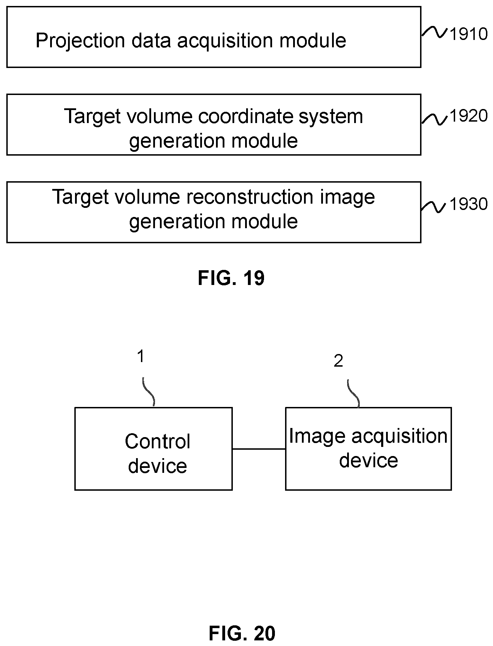

FIG. 19 is a schematic diagram illustrating a structure of a device for generating a volume reconstruction image according to some embodiments of the present disclosure. Referring to FIG. 19, the volume reconstruction image generation device includes: a projection data acquisition module 1910, a target volume coordinate system generation module 1920, and a target volume reconstruction image generation module 1930.

The projection data acquisition module 1910 is configured to obtain projection data at each scanning angle; the target volume coordinate system generation module 1920 is configured to construct a target volume coordinate system based on an initial volume coordinate system and a desired reconstruction direction; the target volume reconstruction image generation module 1930 is configured to reconstruct the projection data according to the desired reconstruction direction under the target volume coordinate system to generate a target volume reconstruction image.

On the basis of each of the above technical solutions, the device further includes: an initial volume coordinate system determination module, wherein, the initial volume coordinate system determination module is used to perform a volume reconstruction of the projection data to obtain an initial volume reconstruction image; determine the initial volume coordinate system based on the initial volume reconstruction image.

On the basis of each of the above technical solutions, a first coordinate original point of the initial volume coordinate system is a point at any position on an edge length of a reconstructed volume at a first longitudinal axis corresponding to the initial volume reconstruction image; a first horizontal axis is in a plane belonging to the first longitudinal axis, starting at the first coordinate original point and perpendicular to the first longitudinal axis; and the first vertical axis starts at the first coordinate original point and is perpendicular to the plane where the first horizontal axis and the first longitudinal axis are located.

On the basis of each of the above technical solutions, the target volume coordinate system generation module 1920 is also used to determine a reconstructed volume to which the initial volume coordinate system belongs;

determine, within the reconstructed volume, a first current plane based on the desired reconstruction direction, in a direction perpendicular to the desired reconstruction direction;

construct a second current plane based on the first longitudinal axis and the first vertical axis, take a midpoint of an intersection of the second current plane and the first current plane as a second coordinate original point of the target volume coordinate system, and take the desired reconstruction direction as a second vertical axis of the target volume coordinate system;

in the first current plane, take an edge length belonging to the second coordinate origin as a second longitudinal axis of the target volume coordinate system, and take a vector starting at the second coordinate original point, perpendicular to the plane in which the second longitudinal axis and the second vertical axis are located, as a second horizontal axis.

On the basis of each of the above technical solutions, the target volume reconstruction image generation module 1930 is further used to determine a pixel range under the target volume coordinate system based on an angular difference between the initial volume coordinate system and the target volume coordinate system, and a pixel range of the initial volume reconstruction image in the initial volume coordinate system;

reconstruct the projection data according to the desired reconstruction direction within the pixel range under the target volume coordinate system to generate the target volume reconstruction image.

On the basis of each of the above technical solutions, the device further includes: a pre-processing module;

wherein the pre-processing module is used to pre-process the projection data.

On the basis of each of the above technical solutions, the device further includes: a rendering module; wherein the rendering module is used to obtain an image rendering instruction of the target volume reconstruction image; render pixel points of the target volume reconstruction image based on the image rendering instruction, and displaying the rendered target volume reconstruction image.

This embodiment provides a technical solution to generate a target volume reconstruction image by obtaining projection data at each scanning angle, constructing a target volume coordinate system based on an initial volume coordinate system and a desired reconstruction direction, and reconstructing the projection data according to the desired reconstruction direction under the target volume coordinate system. The problem that only a better image can be obtained in the direction parallel to the detector but not in other directions in the prior art is solved. By setting different desired reconstruction directions, it achieves the purpose of reconstructing in different desired reconstruction directions and obtaining a better resolution volume reconstruction image in each direction, which facilitates the user to effectively analyze the volume reconstruction images in multiple reconstruction directions and perform the effect of target positioning.

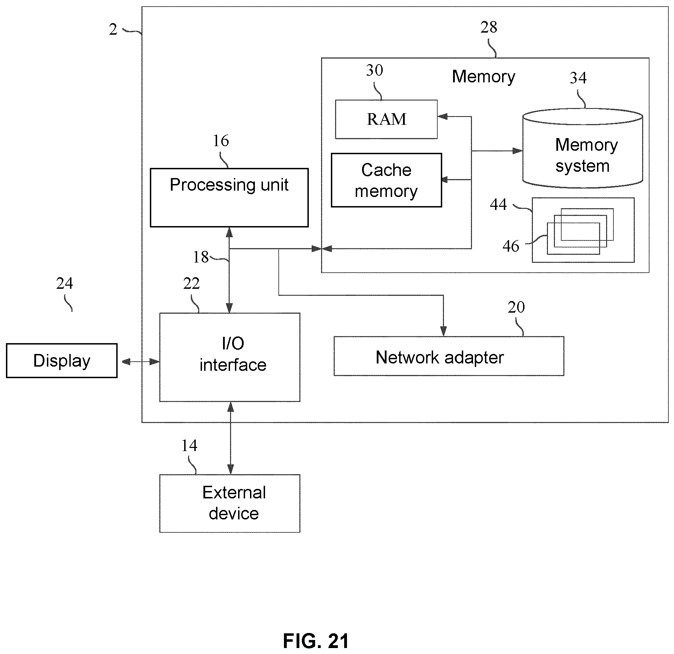

FIG. 20 is a schematic diagram illustrating a structure of a system for generating a volume reconstruction image according to some embodiments of the present disclosure. Referring to FIG. 20, the volume reconstruction image generation system comprises: a control device 1 and an image acquisition device 2, wherein the image acquisition device 2, is used to scan a scanned object at each of the scanning angles to obtain projection data at each of the scanning angles. FIG. 21 shows a block diagram illustrating an exemplary image acquisition device 2 suitable for implementing an implementation of the present disclosure. The image acquisition device 2 shown in FIG. 21 is only an example and shall not impose any limitation on the functionality and scope of use of the embodiments of the present disclosure.

As shown in FIG. 21, the image acquisition device 2 is represented as a general purpose computing device. Components of the image acquisition device 2 may include, but are not limited to: one or more processors or processing units 16, a system memory 28, a bus 18 connecting the different system components including the system memory 28 and the processing units 16.

The bus 18 represents one or more of several types of bus architectures, including memory buses or memory controllers, peripheral buses, graphicals acceleration ports, processors, or local area buses that use any of the multiple bus architectures. Examples of these architectures include, but are not limited to, an Industry Standard Architecture (ISA) bus, a Microchannel Architecture (MAC) bus, an Enhanced ISA bus, a Video Electronics Standards Association (VESA) local area bus, and a Peripheral Component Interconnect (PCI) bus.

The image acquisition device 2 typically includes multiple computer system readable media. These media may be any available media that may be accessible to the image acquisition device 2, including volatile and non-volatile media, removable and non-removable media.

The system memory 28 may include a computer system readable medium in the form of a volatile memory, such as a random access memory (RAM) 30 and/or a cache. The image acquisition device 2 may further include other removable/non-removable, volatile/non-volatile computer system storage media. By way of example only, the memory system 34 may be used to read and write non-removable, non-volatile magnetic media (not shown in FIG. 7, commonly referred to as "hard disk drives"). Although not shown in FIG. 7, the disk drives may be provided for reading and writing to removable non-volatile disks (e.g., "floppy disks"), and optical disk drives for reading and writing to removable non-volatile optical disks (e.g., CD-ROMs, DVD-ROMs, or other optical media). In these cases, each drive may be connected to the bus 18 through one or more data media interfaces. The system memory 28 may include at least one program product having a set of (e.g., the projection data acquisition module 1910 of the volume reconstruction image generation device, the target volume coordinate system generation module 1920, and the target volume reconstruction image generation module 1930) program modules that are configured to perform the functions of the embodiments of the present disclosure.

A program/utility 44 having a set of program modules 46 (e.g., the projection data acquisition module 1910 of the volume reconstruction image generation device, the target volume coordinate system generation module 1920, and the target volume reconstruction image generation module 1930) may be stored, for example, in the system memory 28, such program modules 46 including, but not limited to, an operating system, one or more applications, other program modules, and program data, and each of these examples or some combination may include an implementation of a network environment. The program modules 46 typically perform the functions and/or methods in the embodiments described in the present disclosure.

The image acquisition device 2 may also communicate with one or more external devices 14 (e.g., a keyboard, a pointing device, a display 24, etc.), with one or more devices that enable the user to interact with the image acquisition device 2, and/or with any device that enables the image acquisition device 2 to communicate with one or more other computing devices (e.g., a network card, a modem, etc.). This communication may be via input/output (I/O) interface 22. And, the image acquisition device 2 may also communicate with one or more networks (e.g., a local area network (LAN), a wide area network (WAN), and/or a public network, such as the Internet) through the network adapter 20. As shown, a network adapter 20 communicates with other modules of the image acquisition device 2 via the bus 18. It should be understood that although not shown in the figures, other hardware and/or software modules may be used in conjunction with the image acquisition device 2, including but not limited to: a microcode, a device drive, a redundant processing unit, an external disk drive array, a RAID system, a tape drive, and a data backup storage system, etc.

The processing unit 16 performs various functional applications and data processing by running a program stored in the system memory 28, such as implementing a method for generating a volume reconstruction image as provided in an embodiment of the present disclosure, comprising:

obtaining projection data at each scanning angle;
constructing a target volume coordinate system based on an initial volume coordinate system and a desired reconstruction direction;
reconstructing the projection data according to the desired reconstruction direction under the target volume coordinate system to generate a target volume reconstruction image.

The processing unit 16 performs various functional applications and data processing by running a program stored in the system memory 28, such as implementing a method for generating a volume reconstruction image as provided in an embodiment of the present disclosure.

Of course, it can be understood by those skilled in the art that the processor can also implement the technical solution of a method for generating a volume reconstruction image as provided in any embodiment of the present disclosure.

Embodiments of the present disclosure also provide a computer-readable storage medium having a computer program stored thereon, which when executed by a processor implements a method for generating a volume reconstruction image as provided in embodiments of the present disclosure, comprising:

obtaining projection data at each scanning angle;
constructing a target volume coordinate system based on an initial volume coordinate system and a desired reconstruction direction;
reconstructing the projection data according to the desired reconstruction direction under the target volume coordinate system to generate a target volume reconstruction image. Of course, the computer program stored on the computer-readable storage medium provided in the embodiment of the present disclosure is not limited to the operation of the method as described above, but can also perform the relevant operations in a method for generating a volume reconstruction image provided in any embodiment of the present disclosure.

The computer memory medium of the embodiment of the present disclosure may employ any combination of one or more computer-readable media. The computer-readable medium may be a computer-readable signal medium or a computer-readable memory medium. The computer-readable memory medium may be, for example, but not limited to, an electrical, a magnetic, an optical, an electromagnetic, an infrared, or a semiconductor system, device, or instrument, or any combination of the above. More specific examples of computer-readable memory media (a non-exhaustive list) include: electrically connected with one or more wires, portable computer disks, hard disks, random access memory (RAM), read-only memory (ROM), erasable programmable read-only memory (EPROM or flash memory), optical fiber, portable compact disk read-only memory (CD-ROM), optical memory devices, magnetic memory devices, or any suitable combination of the above. For the purposes of this document, the computer-readable memory medium may be any tangible medium that contains or stores a program that may be used by or in combination with an instruction execution system, device, or instrument.

The computer-readable signal medium may include data signals propagated in a baseband or as part of a carrier wave that carries computer-readable program codes. Such propagated data signals may take multiple forms, including but not limited to an electromagnetic signal, an optical signal, or any suitable combination of the above. The computer-readable signal medium may also be any computer-readable medium other than a computer-readable memory medium that sends, propagates, or transmits a program for use by or in conjunction with an instruction execution system, device, or instrument.

The program codes contained on the computer-readable medium may be transmitted using any suitable medium, including but not limited to a wireless, a wire, a fiber optic cable, RF, etc., or any suitable combination of the above.

The computer program codes for performing the operations of the present disclosure may be written in one or more programming languages or combinations thereof, including object-oriented programming languages such as Java, Smalltalk, C++, and conventional procedural programming languages such as "C" or a similar programming languages. The program codes may be executed entirely on the user's computer, partially on the user's computer, as a stand-alone package, partially on the user's computer and partially on a remote computer, or entirely on a remote computer or server. In the case involving a remote computer, the remote computer may be connected to the user computer via any kind of network including a local area network (LAN) or a wide area network (WAN), or, alternatively, may be connected to an external computer (e.g., using an Internet service provider to connect via the Internet).

It should be noted that the modules included in the above embodiment of the device for generating a volume reconstruction image are only divided according to the functional logic, but are not limited to the above division, as long as they can achieve the corresponding functions; in addition, the specific names of the functional units are only for the purpose of mutual distinction and are not used to limit the scope of protection of the present disclosure.

**Claims**

1. A method for imaging and positioning of a medical imaging device, comprising:

   obtaining a virtual human body model corresponding to an imaging object and first position information input by a user (S310);
   determining, based on the first position information, an internal human body image corresponding to the first position information in the virtual human body model, and displaying the internal human body image (S320);
   determining, based on the first position information, a target imaging position corresponding to the medical imaging device if the internal human body image corresponding to the first position information corresponds to a target photography position (S330);
   if the internal human body image corresponding to the first position information does not correspond to the target photography position, proceeding to obtain second position information different from the first position information and determining the target imaging position corresponding to the medical imaging device based on an internal human body image corresponding to the second position information (S340).

2. The method of claim 1, wherein the first position information includes first model information corresponding to the virtual human body model or first device information corresponding to the medical imaging device, wherein there is an association relationship between the first model information and the first device information.

3. The method of claim 2, wherein the association relationship includes a position association relationship, and the method further includes:
   converting a relative position relationship between the medical imaging device and the imaging object into a position association relationship of the medical imaging device and the virtual human body model; wherein the position association relationship is used to characterize a relationship between position parameters of the first model information and the first device information.

4. The method of claim 3, wherein the association relationship further includes a horizon-of-field association relationship, and the method further includes:
   obtaining a horizon-of-view association relationship between the first model information and the first device information; wherein the horizon-of-view association relationship is used to characterize a relationship between horizon-of-view parameters of the first model information and the first device information.

5. The method of any one of claims 2-4, wherein the obtaining the first position information includes:
   when the first position information is the first model information, displaying the virtual human body model on an interactive interface, and displaying the first model information on the virtual human body model.

6. The method of claim 5, wherein the first model information includes a graphical marker.

7. The method of claim 6, wherein the graphical marker performs at least one operation of selecting, moving, zooming in, and zooming out.

8. The method of any one of claims 2-7, wherein before proceeding to obtain second position information different from the first position information, the method further includes:
   when the first position information is the first model information, determining a first imaging position corresponding to the medical imaging device based on the first model information and the association relationship, and controlling the medical imaging device to move to the first imaging position.

9. The method of any one of claims 2-7, wherein the determining, based on the first position information, a target imaging position corresponding to the medical imaging device includes:
   when the first position information is the first model information, determining a target imaging position corresponding to the medical imaging device based on the first model information and the association relationship, and controlling the medical imaging device to move to the target imaging position.

10. The method of claim 9, wherein after controlling the medical imaging device to move to the target imaging position, the method further includes:
controlling an imaging component in the medical imaging device to perform an imaging operation based on a horizon-of-field parameter; wherein the horizon-of-field parameter includes at least one of a source image distance, a source object distance, and a magnification.

11. The method of any one of claims 2-10, wherein the determining, based on the first position information, an internal human body image corresponding to the first position information in the virtual human body model includes:
determining, when the first position information is the first device information, the first model information corresponding to the virtual human body model based on the first device information and the association relationship, and determining the internal human body image based on the first model information.

12. The method of any one of claims 2-11, wherein the determining, based on the first position information, a target imaging position corresponding to the medical imaging device includes:
when the first position information is the first device information, taking an imaging position in the first device information as the target imaging position corresponding to the medical imaging device.

13. The method of any one of claims 1-12, wherein the obtaining a virtual human body model corresponding to an imaging object includes:
according to obtained height data corresponding to the imaging object, selecting the virtual human body model corresponding to the height data; wherein the virtual human body model includes a human body shape model and an internal human body model.

14. An imaging positioning device for a medical imaging device, comprising:

a virtual human body model acquisition module (1510), configured to obtain a virtual human body model corresponding to an imaging object and obtain first position information input by a user;
an internal human body image display module (1520), configured to determine, based on the first position information, an internal human body image corresponding to the first position information in the virtual human body model, and display the internal human body image;
a first target imaging position determination module (1530), configured to determine, based on the first position information, a target imaging position corresponding to the medical imaging device, if the internal human body image corresponding to the first position information corresponds to a target photography position;
a second target imaging position determination module (1540), configured to proceed to obtain a second position information different from the first position information and determine the target imaging position corresponding to the medical imaging device based on the internal human body image corresponding to the second position information, if the internal human body image corresponding to the first position information does not correspond to the target photography position.

15. A memory medium comprising computer-executable instructions, wherein the computer-executable instructions, when executed by a computer processor, are used to perform an imaging positioning method of a medical imaging device as claimed in any one of claims 1-13.

**Patentansprüche**

1. Verfahren zur Bildgebung und Positionierung einer medizinischen Bildgebungsvorrichtung, umfassend:

Erhalten eines virtuellen Modells eines menschlichen Körpers, das einem Bildgebungsobjekt entspricht, und von ersten Positionsinformationen, die von einem Benutzer eingegeben werden (S310);
Bestimmen, auf Basis der ersten Positionsinformationen, eines Bildes des Inneren des menschlichen Körpers, das den ersten Positionsinformationen entspricht, in dem virtuellen Modell des menschlichen Körpers, und Anzeigen des Bildes des Inneren des menschlichen Körpers (S320);
Bestimmen, auf Basis der ersten Positionsinformationen, einer Ziel-Bildgebungsposition, die der medizinischen Bildgebungsvorrichtung entspricht, wenn das Bild des Inneren des menschlichen Körpers, das den ersten Positionsinformationen entspricht, einer Ziel-Fotografieposition entspricht (S330);

wenn das Bild des Inneren des menschlichen Körpers, das den ersten Positionsinformationen entspricht, nicht der Ziel-Fotografieposition entspricht, Fortfahren, um weite Positionsinformationen zu erhalten, die sich von den ersten Positionsinformationen unterscheiden, und Bestimmen der Ziel-Bildgebungsposition, die der medizinischen Bildgebungsvorrichtung entspricht, auf Basis eines Bildes des Inneren des menschlichen Körpers, das den zweiten Positionsinformationen entspricht (S340).

2. Verfahren nach Anspruch 1, wobei die ersten Positionsinformationen erste Modellinformationen, die dem virtuellen Modell des menschlichen Körpers entsprechen, oder erste Vorrichtungsinformationen beinhalten, die der medizinischen Bildgebungsvorrichtung entsprechen, wobei es eine Zuordnungsbeziehung zwischen den ersten Modellinformationen und den ersten Vorrichtungsinformationen gibt.

3. Verfahren nach Anspruch 2, wobei die Zuordnungsbeziehung eine Positionszuordnungsbeziehung beinhaltet, und das Verfahren weiter beinhaltet:
Umwandeln einer relativen Positionsbeziehung zwischen der medizinischen Bildgebungsvorrichtung und dem Bildgebungsobjekt in eine Positionszuordnungsbeziehung der medizinischen Bildgebungsvorrichtung und des virtuellen Modells des menschlichen Körpers; wobei die Positionszuordnungsbeziehung verwendet wird, um eine Beziehung zwischen Positionsparametern der ersten Modellinformationen und der ersten Vorrichtungsinformationen zu charakterisieren.

4. Verfahren nach Anspruch 3, wobei die Zuordnungsbeziehung weiter eine Feldhorizont-Zuordnungsbeziehung beinhaltet und das Verfahren weiter beinhaltet:
Erhalten einer Sichthorizont-Zuordnungsbeziehung zwischen den ersten Modellinformationen und den ersten Vorrichtungsinformationen; wobei die Sichthorizont-Zuordnungsbeziehung verwendet wird, um eine Beziehung zwischen Sichthorizont-Parametern der ersten Modellinformationen und der ersten Vorrichtungsinformationen zu charakterisieren.

5. Verfahren nach einem der Ansprüche 2-4, wobei das Erhalten der ersten Positionsinformationen beinhaltet:
wenn die ersten Positionsinformationen die ersten Modellinformationen sind, Anzeigen des virtuellen Modells des menschlichen Körpers auf einer interaktiven Schnittstelle, und Anzeigen der ersten Modellinformationen auf dem virtuellen Modell des menschlichen Körpers.

6. Verfahren nach Anspruch 5, wobei die ersten Modellinformationen eine grafische Markierung beinhalten.

7. Verfahren nach Anspruch 6, wobei die grafische Markierung mindestens einen Vorgang von Auswählen, Verschieben, Heranzoomen und Herauszoomen durchführt.

8. Verfahren nach einem der Ansprüche 2-7, wobei das Verfahren vor dem Fortfahren, um zweite Positionsinformationen zu erhalten, die sich von den ersten Positionsinformationen unterscheiden, weiter beinhaltet:
wenn die ersten Positionsinformationen die ersten Modellinformationen sind, Bestimmen einer ersten Bildgebungsposition, die der medizinischen Bildgebungsvorrichtung entspricht, auf Basis der ersten Modellinformationen und der Zuordnungsbeziehung, und Steuern der medizinischen Bildgebungsvorrichtung, um diese zur ersten Bildgebungsposition zu bewegen.

9. Verfahren nach einem der Ansprüche 2-7, wobei das Bestimmen, auf Basis der ersten Positionsinformationen, einer Ziel-Bildgebungsposition, die der medizinischen Bildgebungsvorrichtung entspricht, beinhaltet:
wenn die ersten Positionsinformationen die ersten Modellinformationen sind, Bestimmen einer Ziel-Bildgebungsposition, die der medizinischen Bildgebungsvorrichtung entspricht, auf Basis der ersten Modellinformationen und der Zuordnungsbeziehung, und Steuern der medizinischen Bildgebungsvorrichtung, um diese zur Ziel-Bildgebungsposition zu bewegen.

10. Verfahren nach Anspruch 9, wobei das Verfahren nach dem Steuern der medizinischen Bildgebungsvorrichtung, um diese zur Ziel-Bildgebungsposition zu bewegen, weiter beinhaltet:
Steuern einer Bildgebungskomponente in der medizinischen Bildgebungsvorrichtung, um einen Bildgebungsvorgang durchzuführen, auf Basis eines Feldhorizontparameters; wobei der Feldhorizontparameter mindestens eines von einem Quelle-Bild-Abstand, einem Quelle-Objekt-Abstand und einer Vergrößerung beinhaltet.

11. Verfahren nach einem der Ansprüche 2-10, wobei das Bestimmen, auf Basis der ersten Positionsinformationen, eines Bildes des Inneren des menschlichen Körpers, das den ersten Positionsinformationen entspricht, im virtuellen Modell des menschlichen Körpers beinhaltet:

wenn die ersten Positionsinformationen die ersten Vorrichtungsinformationen sind, Bestimmen der ersten Modellinformationen, die dem virtuellen Modell des menschlichen Körpers entsprechen, auf Basis der ersten Vorrichtungsinformationen und der Zuordnungsbeziehung, und Bestimmen des Bildes des Inneren des menschlichen Körpers auf Basis der ersten Modellinformationen.

12. Verfahren nach einem der Ansprüche 2-11, wobei das Bestimmen, auf Basis der ersten Positionsinformationen, einer Ziel-Bildgebungsposition, die der medizinischen Bildgebungsvorrichtung entspricht, beinhaltet:

wenn die ersten Positionsinformationen die ersten Vorrichtungsinformationen sind, Heranziehen einer Bildgebungsposition in den ersten Vorrichtungsinformationen als Ziel-Bildgebungsposition, die der medizinischen Bildgebungsvorrichtung entspricht.

13. Verfahren nach einem der Ansprüche 1-12, wobei das Erhalten eines virtuellen Modells eines menschlichen Körpers, das einem Bildgebungsobjekt entspricht, beinhaltet:

Auswählen, gemäß erhaltenen Höhendaten, die dem Bildgebungsobjekt entsprechen, des virtuellen Modells des menschlichen Körpers, das den Höhendaten entspricht; wobei das virtuelle Modell des menschlichen Körpers ein Modell der menschlichen Körperform und ein Modell des Inneren des menschlichen Körpers beinhaltet.

14. Bildgebungspositionierungsvorrichtung für eine medizinische Bildgebungsvorrichtung, umfassend:

ein Modul zum Erfassen (1510) eines virtuellen Modells eines menschlichen Körpers, das dazu konfiguriert ist, ein virtuelles Modell eines menschlichen Körpers, das einem Bildgebungsobjekt entspricht, zu erhalten, und erste Positionsinformationen, die von einem Benutzer eingegeben werden, zu erhalten;

ein Modul zum Anzeigen (1520) eines Bildes des Inneren des menschlichen Körpers, das dazu konfiguriert ist, auf Basis der ersten Positionsinformationen ein Bild des Inneren des menschlichen Körpers, das den ersten Positionsinformationen entspricht, in dem virtuellen Modell des menschlichen Körpers zu bestimmen, und das Bild des Inneren des menschlichen Körpers anzuzeigen;

ein erstes Modul zum Bestimmen (1530) einer Ziel-Bildgebungsposition, das dazu konfiguriert ist, auf Basis der ersten Positionsinformationen eine Ziel-Bildgebungsposition zu bestimmen, die der medizinischen Bildgebungsvorrichtung entspricht, wenn das Bild des Inneren des menschlichen Körpers, das den ersten Positionsinformationen entspricht, einer Ziel-Fotografieposition entspricht;

ein zweites Modul zum Bestimmen (1540) einer Ziel-Bildgebungsposition, das dazu konfiguriert ist, fortzufahren, um zweite Positionsinformationen zu erhalten, die sich von den ersten Positionsinformationen unterscheiden, und die Ziel-Bildgebungsposition, die der medizinischen Bildgebungsvorrichtung entspricht, auf Basis des Bildes des Inneren des menschlichen Körpers, das den zweiten Positionsinformationen entspricht, zu bestimmen, wenn das Bild des Inneren des menschlichen Körpers, das den ersten Positionsinformationen entspricht, nicht der Ziel-Fotografieposition entspricht.

15. Speichermedium, das computerausführbare Anweisungen umfasst, wobei die computerausführbaren Anweisungen, wenn sie von einem Computerprozessor ausgeführt werden, verwendet werden, um ein Bildgebungspositionierungsverfahren einer medizinischen Bildgebungsvorrichtung nach einem der Ansprüche 1-13 durchzuführen.

## Revendications

1. Procédé d'imagerie et de positionnement d'un dispositif d'imagerie médicale, comprenant :

l'obtention d'un modèle virtuel de corps humain correspondant à un objet d'imagerie et de premières informations de position saisies par un utilisateur (S310) ;

la détermination, sur la base des premières informations de position, d'une image interne de corps humain correspondant aux premières informations de position dans le modèle virtuel de corps humain, et l'affichage de l'image interne de corps humain (S320) ;

la détermination, sur la base des premières informations de position, d'une position d'imagerie cible correspondant au dispositif d'imagerie médicale si l'image interne de corps humain correspondant aux premières informations de position correspond à une position de photographie cible (S330) ;

si l'image interne de corps humain correspondant aux premières informations de position ne correspond pas à la position de photographie cible, le passage à l'obtention de secondes informations de position différentes des premières informations de position et la détermination de la position d'imagerie cible correspondant au dispositif d'imagerie médicale sur la base d'une image interne de corps humain correspondant aux secondes informations de position (S340).

2. Procédé selon la revendication 1, dans lequel les premières informations de position incluent des premières informations de modèle correspondant au modèle virtuel de corps humain ou des premières informations de dispositif correspondant au dispositif d'imagerie médicale, dans lequel il existe une relation d'association entre les premières informations de modèle et les premières informations de dispositif.

3. Procédé selon la revendication 2, dans lequel la relation d'association inclut une relation d'association de position, et le procédé inclut en outre :
la conversion d'une relation de position relative entre le dispositif d'imagerie médicale et l'objet d'imagerie en une relation d'association de position du dispositif d'imagerie médicale et du modèle virtuel de corps humain ; dans lequel la relation d'association de position est utilisée pour caractériser une relation entre des paramètres de position des premières informations de modèle et des premières informations de dispositif.

4. Procédé selon la revendication 3, dans lequel la relation d'association inclut en outre une relation d'association d'horizon de champ, et le procédé inclut en outre :
l'obtention d'une relation d'association d'horizon de vue entre les premières informations de modèle et les premières informations de dispositif; dans lequel la relation d'association d'horizon de vue est utilisée pour caractériser une relation entre des paramètres d'horizon de vue des premières informations de modèle et des premières informations de dispositif.

5. Procédé selon l'une quelconque des revendications 2-4, dans lequel l'obtention des premières informations de position inclut :
lorsque les premières informations de position sont les premières informations de modèle, l'affichage du modèle virtuel de corps humain sur une interface interactive et l'affichage des premières informations de modèle sur le modèle virtuel de corps humain.

6. Procédé selon la revendication 5, dans lequel les premières informations de modèle incluent un marqueur graphique.

7. Procédé selon la revendication 6, dans lequel le marqueur graphique effectue au moins une opération de sélection, de déplacement, de zoom avant et de zoom arrière.

8. Procédé selon l'une quelconque des revendications 2-7, dans lequel, avant de passer à l'obtention de secondes informations de position différentes des premières informations de position, le procédé inclut en outre :
lorsque les premières informations de position sont les premières informations de modèle, la détermination d'une première position d'imagerie correspondant au dispositif d'imagerie médicale sur la base des premières informations de modèle et de la relation d'association, et la commande du dispositif d'imagerie médicale pour qu'il se déplace vers la première position d'imagerie.

9. Procédé selon l'une quelconque des revendications 2-7, dans lequel la détermination, sur la base des premières informations de position, d'une position d'imagerie cible correspondant au dispositif d'imagerie médicale inclut :
lorsque les premières informations de position sont les premières informations de modèle, la détermination d'une position d'imagerie cible correspondant au dispositif d'imagerie médicale sur la base des premières informations de modèle et de la relation d'association, et la commande du dispositif d'imagerie médicale pour qu'il se déplace vers la position d'imagerie cible.

10. Procédé selon la revendication 9, dans lequel, après la commande du dispositif d'imagerie médicale pour qu'il se déplace vers la position d'imagerie cible, le procédé inclut en outre :
la commande d'un composant d'imagerie dans le dispositif d'imagerie médicale pour qu'il effectue une opération d'imagerie sur la base d'un paramètre d'horizon de champ ; dans lequel le paramètre d'horizon de champ inclut au moins l'une d'une distance image-source, d'une distance objet-source et d'un grossissement.

11. Procédé selon l'une quelconque des revendications 2-10, dans lequel la détermination, sur la base des premières informations de position, d'une image interne de corps humain correspondant aux premières informations de position dans le modèle virtuel de corps humain inclut :
la détermination, lorsque les premières informations de position sont les premières informations de dispositif, des premières informations de modèle correspondant au modèle virtuel de corps humain sur la base des premières informations de dispositif et de la relation d'association, et la détermination de l'image interne de corps humain sur la base des premières informations de modèle.

12. Procédé selon l'une quelconque des revendications 2-11, dans lequel la détermination, sur la base des premières informations de position, d'une position d'imagerie cible correspondant au dispositif d'imagerie médicale inclut :
lorsque les premières informations de position sont les premières informations de dispositif, le fait de prendre une position d'imagerie dans les premières informations de dispositif en tant que position d'imagerie cible correspondant au dispositif d'imagerie médicale.

13. Procédé selon l'une quelconque des revendications 1-12, dans lequel l'obtention d'un modèle virtuel de corps humain correspondant à un objet d'imagerie inclut :
selon des données de hauteur obtenues correspondant à l'objet d'imagerie, la sélection du modèle virtuel de corps humain correspondant aux données de hauteur ; dans lequel le modèle virtuel de corps humain inclut un modèle de forme de corps humain et un modèle de corps humain interne.

14. Dispositif de positionnement d'imagerie pour un dispositif d'imagerie médicale, comprenant :

un module d'acquisition de modèle virtuel de corps humain (1510), configuré pour obtenir un modèle virtuel de corps humain correspondant à un objet d'imagerie et obtenir des premières informations de position saisies par un utilisateur ;
un module d'affichage d'image interne de corps humain (1520), configuré pour déterminer, sur la base des premières informations de position, une image interne de corps humain correspondant aux premières informations de position dans le modèle virtuel de corps humain, et afficher l'image interne de corps humain ;
un premier module de détermination de position d'imagerie cible (1530), configuré pour déterminer, sur la base des premières informations de position, une position d'imagerie cible correspondant au dispositif d'imagerie médicale, si l'image interne de corps humain correspondant aux premières informations de position correspond à une position de photographie cible ;
un second module de détermination de position d'imagerie cible (1540), configuré pour passer à l'obtention de secondes informations de position différentes des premières informations de position et déterminer la position d'imagerie cible correspondant au dispositif d'imagerie médicale sur la base de l'image interne de corps humain correspondant aux secondes informations de position, si l'image interne de corps humain correspondant aux premières informations de position ne correspond pas à la position de photographie cible.

15. Support mémoire comprenant des instructions exécutables par ordinateur, dans lequel les instructions exécutables par ordinateur, lorsqu'elles sont exécutées par un processeur informatique, sont utilisées pour mettre en œuvre un procédé de positionnement d'imagerie d'un dispositif d'imagerie médicale selon l'une quelconque des revendications 1-13.

<u>100</u>

110

120

140

150

Memory
device

Network

130

131    132    133

···

FIG. 1

200

Photographing a subject during a photography cycle, obtaining, during the photography cycle, first fluoroscopic data of a radiation source irradiating the subject at a first energy, and obtain second fluoroscopic data of the radiation source irradiating the subject at a second energy different from the first energy
210

Performing the photography of the subject in multiple successive photography cycles
220

Displaying a dynamic image of the subject based on the first fluoroscopic data and the second fluoroscopic data obtained in each of the multiple successive photography cycles
230

**FIG. 2**

Obtaining a virtual human body model corresponding to an imaging object and obtaining first position information corresponding to a user operation instruction ~ S310

Based on the first position information, determining an internal human body image corresponding to the first position information in the virtual human body model, and displaying the internal human body image ~ S320

If the first position information corresponds to the internal human body image and the target photography position, determining a target imaging position corresponding to the medical imaging device based on the first position information ~ S330

If the internal human body image corresponding to the first position information does not correspond to the target photography position, proceeding to obtain a second position information different from the first position information, and determining a target imaging position corresponding to the medical imaging device based on the internal human body image corresponding to the second position information ~ S340

**FIG. 3**

Obtaining a virtual human body model corresponding to an imaging object — S410

Displaying the virtual human model on an interactive interface, and displaying first model information corresponding to the user operation instruction on the virtual human model — S420

Based on the first model information, determining an internal human body image corresponding to the first model information in the virtual human body model, and displaying the internal human body image — S430

Determining, based on the first model information, a target imaging position corresponding to the medical imaging device, if the internal human body image corresponding to the first model information corresponds to a target photography position — S440

If the internal human body image corresponding to the first model information does not correspond to the target photography position, proceeding to obtain second model information different from the first position information and determining the target imaging position corresponding to the medical imaging device based on the internal human body image corresponding to the second model information — S450

**FIG. 4**

FIG. 5

| Obtaining a virtual human body model corresponding to an imaging object, and obtaining first device information corresponding to a user operation instruction | S610 |

| Determining first model information corresponding to the virtual human body model based on the first device information and the association relationship, and determining an internal human body image based on the first model information | S620 |

| Determining, based on the first position information, a target imaging position corresponding to the medical imaging device, if the internal human body image corresponding to the first device information corresponds to a target photography position | S630 |

| If the internal human body image corresponding to the first device information does not correspond to the target photography position, proceeding to obtain second device information different from the first device information and determining the target imaging position corresponding to the medical imaging device based on the internal human body image corresponding to the second device information | S640 |

**FIG. 6**

Treatment bed

Virtual human body model

Radiation source

**FIG. 7**

Obtaining a first blood vessel image of an imaging object obtained by the angiography device based on a first horizon-of-view parameter, and displaying the first blood vessel image — S810

Determining a second horizon-of-view parameter based on a received parameter adjustment instruction and the first horizon-of-view parameter — S820

Determining a second blood vessel image based on the second horizon-of-view parameter and simultaneously displaying the second blood vessel image and the first blood vessel image — S830

**FIG. 8**

Obtaining a first blood vessel image of an imaging object obtained by the angiography device based on a first horizon-of-view parameter, and displaying the first blood vessel image ⎯⏜⏜ S910

Determining a second horizon-of-view parameter based on a received parameter adjustment instruction and the first horizon-of-view parameter, and determining a second blood vessel image based on the second horizon-of-view parameter ⎯⏜⏜ S920

Displaying the second blood vessel image simultaneously with the first blood vessel image ⎯⏜⏜ S930

Obtaining an updated first blood vessel image obtained by the angiography device based on a changed first horizon-of-view parameter when the first blood vessel image corresponds to a change in the first horizon-of-view parameter ⎯⏜⏜ S940

Determining an updated second horizon-of-view parameter based on the changed first horizon-of-view parameter and the parameter adjustment instruction ⎯⏜⏜ S950

Simultaneously displaying the updated second blood vessel image determined based on the updated second horizon-of-view parameter and the updated first blood vessel image ⎯⏜⏜ S960

**FIG. 9**

Obtaining projection data at each scanning angle — S1010

Constructing a target volume coordinate system based on an initial volume coordinate system and a desired reconstruction direction — S1020

in the target volume coordinate system, reconstructing the projection data according to the desired reconstruction direction to generate a target volume reconstruction image — S1030

**FIG. 10**

Reconstruction volume

z

x

Volume Z

Volume X

O    Volume Y

y

**FIG. 11**

**FIG. 12**

---

| Obtaining projection data at each scanning angle | ∿ S1310 |

↓

| Constructing a target volume coordinate system based on an initial volume coordinate system and a desired reconstruction direction | ∿ S1320 |

↓

| Determining a pixel range under the target volume coordinate system based on an angular difference between the initial volume coordinate system and the target volume coordinate system, and a pixel range of the initial volume reconstruction image in the initial volume coordinate system | ∿ S1330 |

↓

| In the pixel range under the target volume coordinate system, reconstructing the projection data according to the desired reconstruction direction to generate a target volume reconstruction image | ∿ S1340 |

**FIG. 13**

300

Photography module
1410

Display module 1420

**FIG. 14**

Virtual human body model
acquisition module — 1510

Internal human body image display
module — 1520

First target imaging position
determination module — 1530

Second target imaging position
determination module — 1540

**FIG. 15**

16

Memory

1621

162

RAM

1623

Memory
system

161

Cache
memory

1622

1625

1624

Processing
unit

163

1641

165

166

Display

I/O
interface

Network adapter

164

External
device

**FIG. 16**

First blood vessel image display
module

1710

Second horizon-of-view parameter
determination module

1720

Second blood vessel image display
module

1730

**FIG. 17**

182

Imaging
assembly

180

Controller

Display device

181

**FIG. 18**

| Projection data acquisition module | 1910 |

| Target volume coordinate system generation module | 1920 |

| Target volume reconstruction image generation module | 1930 |

**FIG. 19**

1

2

| Control device | Image acquisition device |

**FIG. 20**

**FIG. 21**

**EP 4 201 331 B1**

**REFERENCES CITED IN THE DESCRIPTION**

**Patent documents cited in the description**

- US 2016287201 A1